(19) Europäisches Patentamt
European Patent Office
Office européen des brevets

(11) Veröffentlichungsnummer : **0 397 175 B1**

(12) # EUROPÄISCHE PATENTSCHRIFT

(45) Veröffentlichungstag der Patentschrift : **08.12.93 Patentblatt 93/49**

(21) Anmeldenummer : **90108827.8**

(22) Anmeldetag : **10.05.90**

(51) Int. Cl.$^5$ : **C07D 487/04**, C07D 207/337, A61K 31/40, // (C07D487/04, 209:00, 209:00)

(54) **Substituierte Pyrrolizinverbindungen und deren Anwendung in der Pharmazie.**

Die Akte enthält technische Angaben, die nach dem Eingang der Anmeldung eingereicht wurden und die nicht in dieser Patentschrift enthalten sind.

(30) Priorität : **11.05.89 DE 3915450**

(43) Veröffentlichungstag der Anmeldung : **14.11.90 Patentblatt 90/46**

(45) Bekanntmachung des Hinweises auf die Patenterteilung : **08.12.93 Patentblatt 93/49**

(84) Benannte Vertragsstaaten : **AT BE CH DE DK ES FR GB GR IT LI LU NL SE**

(56) Entgegenhaltungen :
**WO-A-82/02044**
**DE-A- 1 940 551**
**DE-A- 2 062 984**
**DE-A- 2 261 965**
**GB-A- 1 156 477**
**US-A- 4 284 562**
**ARCHIV DER PHARMAZIE, Band 321, Seiten 159-162, 1988; G. DANNHARDT et al.: "Antiphlogistische 2,3-Dihydro-1H-pyrrolizine, 13. Mitt. - Stellungsisomere Diaryldihydropyrrolizinyl-essigsäuren und -hydroxyethyl-Derivate"**
**ARCHIV DER PHARMAZIE, Band 321, Seiten 545-549, 1988; G. DANNHARDT et al.: "Antiphlogistische 2,3-Dihydro-1H-pyrrolizine, 14. Mitt. - Stellungsisomere Diaryldihydropyrrolizinyl-ameisensäuren und -propionsäuren"**
**CHEMIKER-ZEITUNG, 110. Jahrgang, Seiten 267-271, 1986; G. DANNHARDT et al.: "Antiphlogistische 2,3-Dihydro-1H-pyrrolizine, 12. Mitt.[1] - Carbaldehyde und hydroxymethylierte Derivate stellungsisomerer Diaryl-2,3-dihydro-1H-pyrrolizine (5,6-,5,7- und 6,7-DADHP-Derivate)"**

(56) Entgegenhaltungen :
**ARCHIV DER PHARMAZIE, Band 312, Seiten 896-907, 1979; G. DANNHARDT et al.: "Synthese und Eigenschaften von 2,3-Dihydro-1H-pyrrolizinen"**

(73) Patentinhaber : **Dannhardt, Gerd, Prof.Dr.**
**Königsbergerstrasse 26a**
**D-65760 Eschborn (DE)**
Patentinhaber : **MERCKLE GMBH**
**Graf-Arco-Strasse 3**
**D-89079 Ulm (DE)**

(72) Erfinder : **Dannhardt, Gerd, Prof. Dr.**
**Königsbergerstrasse 26a**
**W-6236 Eschborn 2 (DE)**
Erfinder : **Steindl, Ludwig, Dr.**
**Rehbergstrasse 5a**
**W-8000 München 71 (DE)**
Erfinder : **Lehr, Matthias, Dr.**
**Heiglhofstrasse 111**
**W-8000 München 70 (DE)**
Erfinder : **Laufer, Stefan, Dr.**
**Webergasse 9**
**W-7902 Blaubeuren (DE)**

(74) Vertreter : **Kinzebach, Werner, Dr. et al**
**Patentanwälte Reitstötter, Kinzebach und Partner Sternwartstrasse 4 Postfach 86 06 49**
**D-81633 München (DE)**

EP 0 397 175 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft substituierte Pyrrolizinverbindungen und deren Anwendung in der Pharmazie sowie pharmazeutische Mittel, welche diese Verbindungen enthalten.

Es ist bekannt, daß die Metabolisierung von Arachidonsäure auf zwei verschiedenen Wegen erfolgt. Beim Cyclooxygenase-Weg wird unter Einwirkung des Enzyms Cyclooxygenase die Arachidonsäure zu Prostaglandinen metabolisiert. Beim Lipoxygenase-Weg wird die Arachidonsäure unter Einwirkung von Lipoxygenasen zu den sogenannten Leukotrienen metabolisiert.

Die Prostaglandine sind an der Entstehung von Entzündung, Fieber und Schmerz beteiligt, während die Leukotriene bei der Entstehung von Asthma, Entzündungen und Allergien eine wichtige Rolle spielen. Zur Bekämpfung dieser Symptome werden häufig nicht-steroidale Antiphlogistica, wie Arylessigsäure-, 2-Arylpropionsäure- und Anthranilsäure-Derivate, eingesetzt. Diese Derivate hemmen die Cyclooxygenase und verhindern somit die Bildung der Prostaglandine aus Arachidonsäure. Die Anwendung derartiger Derivate ist jedoch aufgrund ihrer Nebenwirkungen nicht unbedenklich. Arzneimittel, welche die Lipoxygenase hemmen, sind im Handel jedoch nicht erhältlich.

In Archiv der Pharmazie, 312,896-907 (1979); 321, 159-162 (1988); 321, 545-549 (1988) sind substituierte 2,3-Dihydro-1H-pyrrolizinverbindungen und ihre Herstellung beschrieben. Diese Verbindungen weisen in 5-, 6- und 7-Stellung Substituenten auf, wobei zwei davon eine unsubstituierte Phenylgruppe sind.

In Chemikerzeitung , 110,267-271 (1986) sind ebenfalls substuierte 2,3-Dihydro-1 H-pyrrolizinverbindungen der oben beschriebenen Art offenbart. Bei zwei der Substituenten in 5-, 6- und 7-Stellung handelt es sich um chlor- bzw. methoxy-substitierte Phenylgruppen. Der dritte Substituent ist eine Carbaldehydgruppe oder eine Hydroxymethylgruppe.

Die DE-A-1940551 beschreibt Dihydropyrrolizinverbindungen der Formel:

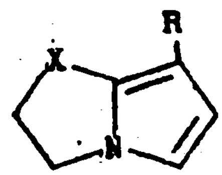

worin

X u.a. eine Methylengruppe bedeutet und R für eine Hydroxymethylgruppe oder eine Formylgruppe steht. Diese Verbindungen besitzen Antivirusaktivität, Antitumoraktivität und immunosuppressive Aktivität.

Überraschenderweise wurde nun gefunden, daß bestimmte substituierte Pyrrolverbindungen potente Cyclooxygenase- und/ oder Lipoxygenasehemmer sind und daher zur Prävention von allergisch induzierten Erkrankungen und zur Behandlung von Erkrankungen des rheumatischen Formenkreises brauchbar sind.

Gegenstand der Erfindung sind daher substituierte Pyrrolizinverbindungen der allgemeinen Formel I:

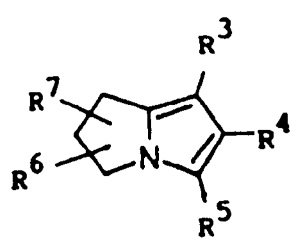

I

worin

jeweils zwei der Reste $R^3$, $R^4$ und $R^5$ unabhängig voneinander eine Arylgruppe, die gegebenenfalls durch einen oder zwei Reste substituiert ist, die ausgewählt sind unter einem Halogenatom, einer Nitro-, $C_1$-$C_4$-Alkoxy-, Hydroxy-, $C_1$-$C_4$-Alkyl- oder Phenoxygruppe

bedeuten, aber nicht gleichzeitig für eine unsubstituierte Arylgruppe stehen, und

der Dritte der Reste $R^3$, $R^4$ und $R^5$ für $-CO_2H$, $-COSC_1$-$C_4$-Alkyl oder A-X steht, wobei A eine geradkettige oder verzweigte $C_1$-$C_8$-Alkylengruppe, die gegebenenfalls durch ein Sauerstoffheteroatom oder eine Carbonylgruppe unterbrochen sein kann, oder eine $C_2$-$C_8$ Alkenylengruppe bedeutet und X für $CO_2H$, $SO_3H$, CHO, oder SH steht, und

$R^6$ und $R^7$ unabhängig voneinander für ein Wasserstoffatom oder eine $C_1$-$C_4$-Alkylgruppe stehen,

sowie deren pharmazeutisch verträgliche Salze und Ester.

Die pharmazeutisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Milchsäure, Citronensäure, Äpfelsäure, Mandelsäure, Ascorbinsäure, Maleinsäure, Fumarsäure, Gluconsäure und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Zu den Estern der Verbindungen der Formel I zählen insbesondere physiologisch leicht hydrolysierbare Ester, beispielsweise Alkyl-, Pivaloyloxymethyl-, Acetoxymethyl-, Phthalidyl-, Indanyl- und Methoxymethylester.

Der Ausdruck "Alkyl, Alkoxy etc." umfaßt geradkettige oder verzweigte Alkylgruppen, wie Methyl, Ethyl, n- und i-Propyl, n-, i- oder t-Butyl, n-Pentyl, Neopenthyl, n-Hexyl etc.

Soweit nicht anders angegeben, steht "Alkyl" vorzugsweise für $C_1$-$C_8$-Alkyl und insbesondere für $C_1$-$C_6$-Alkyl.

Aryl steht vorzugsweise für Naphthyl und insbesondere für Phenyl.

Der Ausdruck "Halogenatom" umfaßt ein Fluor-, Chlor-, Brom- oder Jodatom und insbesondere ein Fluor- oder Chloratom.

Der Cycloalkylrest steht vorzugsweise für einen Cyclopentyl oder Cyclohexylrest.

Besonders bevorzugt sind Verbindungen der Formel I, worin zwei der Reste $R^3$, $R^4$ und $R^5$ für eine Phenylgruppe, die
gegebenenfalls substituiert ist durch ein oder zwei Reste, die ausgewählt sind unter einem Halogenatom, insbesondere ein Fluor- oder Chloratom, einer $C_1$-$C_4$-Alkyl-, $C_1$-$C_4$-Alkoxy-, Hydroxy- und Phenoxygruppe, stehen und der dritte Rest für A-X steht, wobei A eine $C_1$-$C_8$-Alkylengruppe oder eine $C_2$-$C_8$-Alkenylengruppe bedeutet und X für $CO_2H$ steht.

AX steht insbesondere für $C_2$-$C_6$-Alkylen-$CO_2H$ oder $C_2$-$C_6$-Alkenylen-$CO_2H$ und besonders bevorzugt für $(CH_2)_2CO_2H$ , $CH_2CO_2H$ oder $CH{=}CH\ CO_2H$.

Vorzugsweise stehen $R^6$ und $R^7$ in 2-Stellung des Pyrrolizingerüstes und insbesondere bedeuten beide ein Wasserstoffatom oder eine Methylgruppe.

Besonders bevorzugte Ausführungsformen sind die Verbindungen der Formeln:

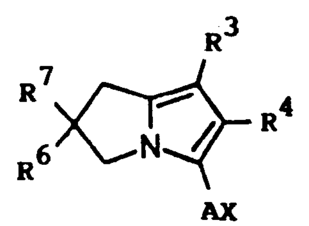

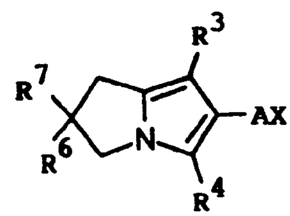

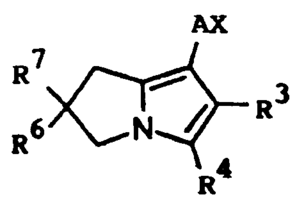

worin $R^6$ und $R^7$ ein Wasserstoffatom oder eine $C_1$-$C_4$ Alkylgruppe bedeuten und
$R^3$ und $R^4$ eine Phenylgruppe bedeuten, die gegebenenfalls durch ein oder zwei Reste substituiert ist, die ausgewählt sind unter einem Halogenatom, einer Nitro-, $C_1$-$C_4$-Alkoxy-, $C_1$-$C_4$-Alkyl- oder Phenoxygruppe. A-X besitzt die oben im Zusammenhang mit der Formel I angegebenen Bedeutungen.

Die Substituenten der Phenylgruppe sind dabei vorzugsweise ausgewählt unter einem Halogenatom, insbesondere einem Fluor- oder Chloratom, einer $C_1$-$C_4$-Alkyl-, $C_1$-$C_4$-Alkoxy- und Phenoxygruppe. Die Substituenten befinden sich vorzugsweise in m- und/oder p-Stellung.

A steht dabei vorzugsweise für eine $C_1$-$C_8$-Alkylengruppe oder $C_2$-$C_8$Alkenylengruppe, insbesondere für eine $C_2$-$C_6$-Alkylen- oder $C_2$-$C_6$-Alkenylengruppe, X steht vorzugsweise für $CO_2H$.

Eine besonders bevorzugte Verbindungen ist:

3-(2,2-Dimethyl-6-(4-phenoxyphenyl)-7-phenyl-2,3-dihydro-1H-pyrrolizin-5-yl) propionsäure.

Die Synthese der Verbindungen der Formel I erfolgt in einem ersten Schritt gemäß der nachfolgenden Gleichung (1):

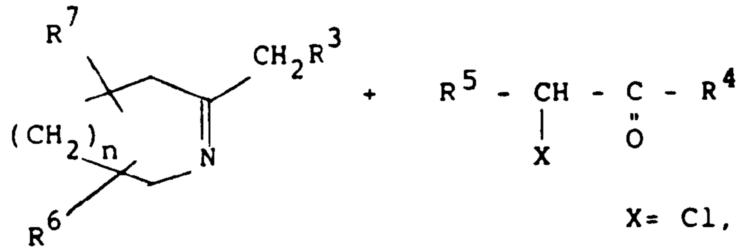

X= Cl, Br

n = 1

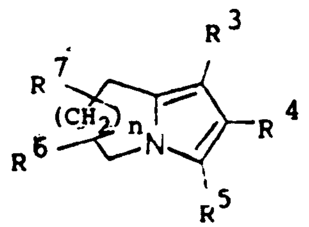

Dieser Syntheseschritt wird ebenso wie die nachfolgenden am Beispiel der diphenylsubstituierten Pyrrolizinverbindungen erläutert. Der erste Reaktionsschritt ist in dem nachfolgenden Reaktionsschema näher erläutert:

Reaktionsschema (1):

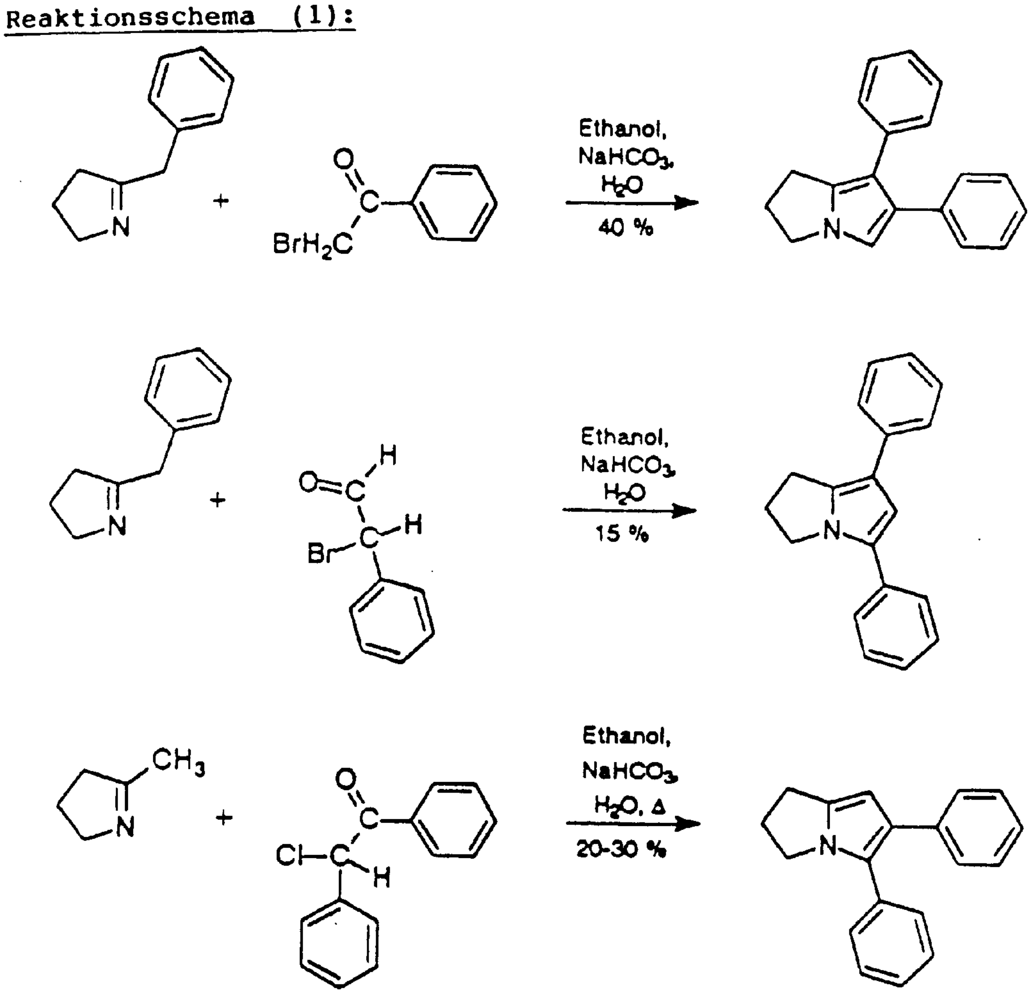

Die Reaktionsbedingungen für diese Umsetzungen sind bekannt, sie sind beschrieben in Chemiker-Zeitung, 110 (1986), Nr. 7/8, Seiten 267, 271 und Arch. Pharm. 321, 159 bis 162 (1988).

In einem zweiten Schritt erfolgt die Einführung einer Formyl- oder Essigestergruppe in den Pyrrolring gemäß dem nachfolgenden Reaktionsschema (2):

Reaktionsschema (2):

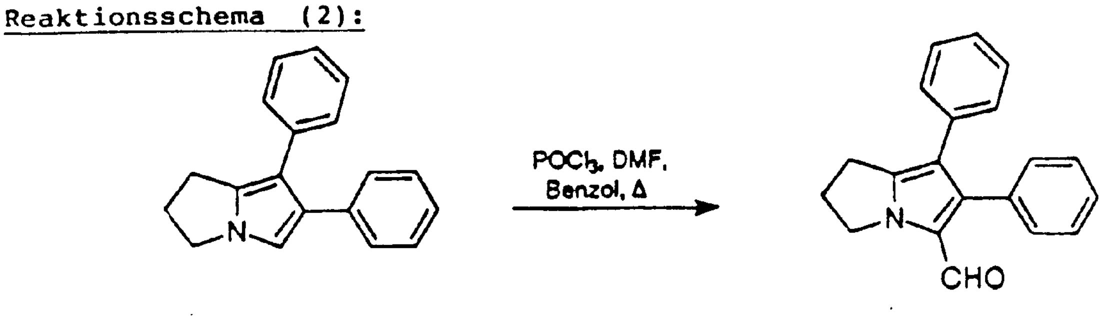


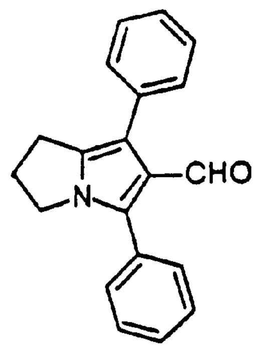

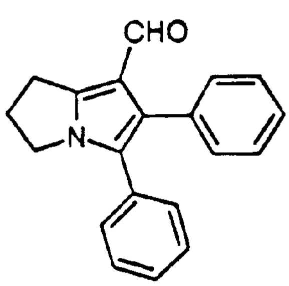

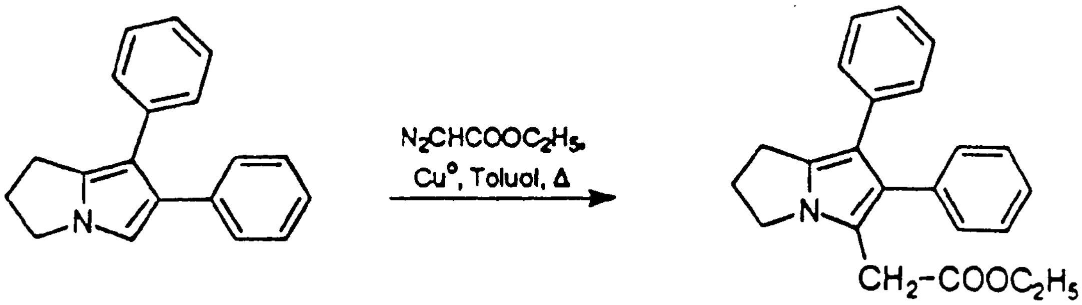


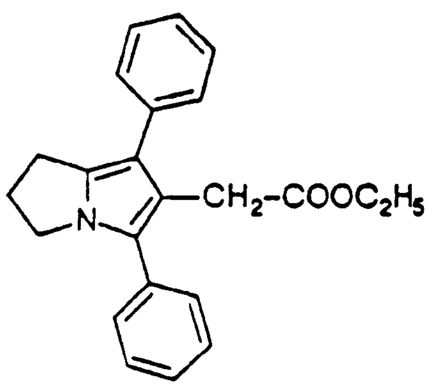

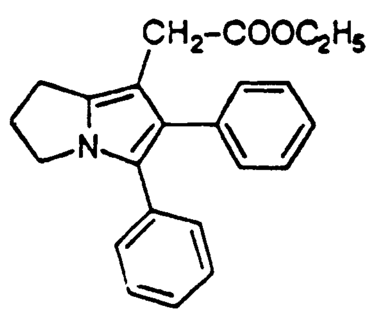

Diese Umsetzungen und die Reaktionsbedingungen sind bekannt, sie sind beschrieben in Chemiker-Zeitung, 110 (1986), Nr. 7/8, 267 bis 271 und Arch. Pharm. 321, 159 bis 162 (1988).

Die Herstellung der entsprechenden Ameisensäure-, Propionsäure- und Acrylsäurederivate ist im Arch. Pharm. 321, 545 bis 549 beschrieben. Sie erfolgt gemäß den folgenden Reaktionsschemata (3) und (4):

Reaktionsschema (3):

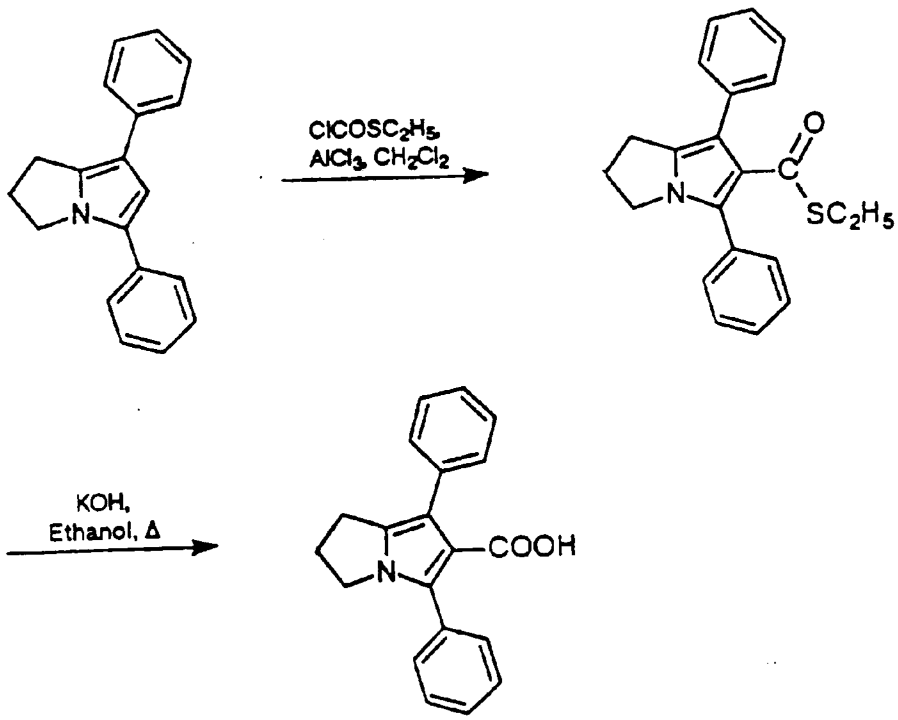


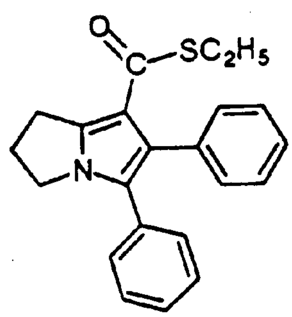

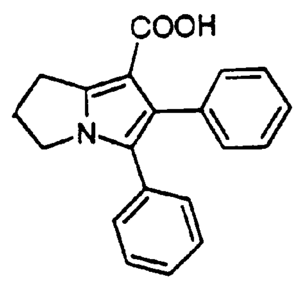

Reaktionsschema (4):

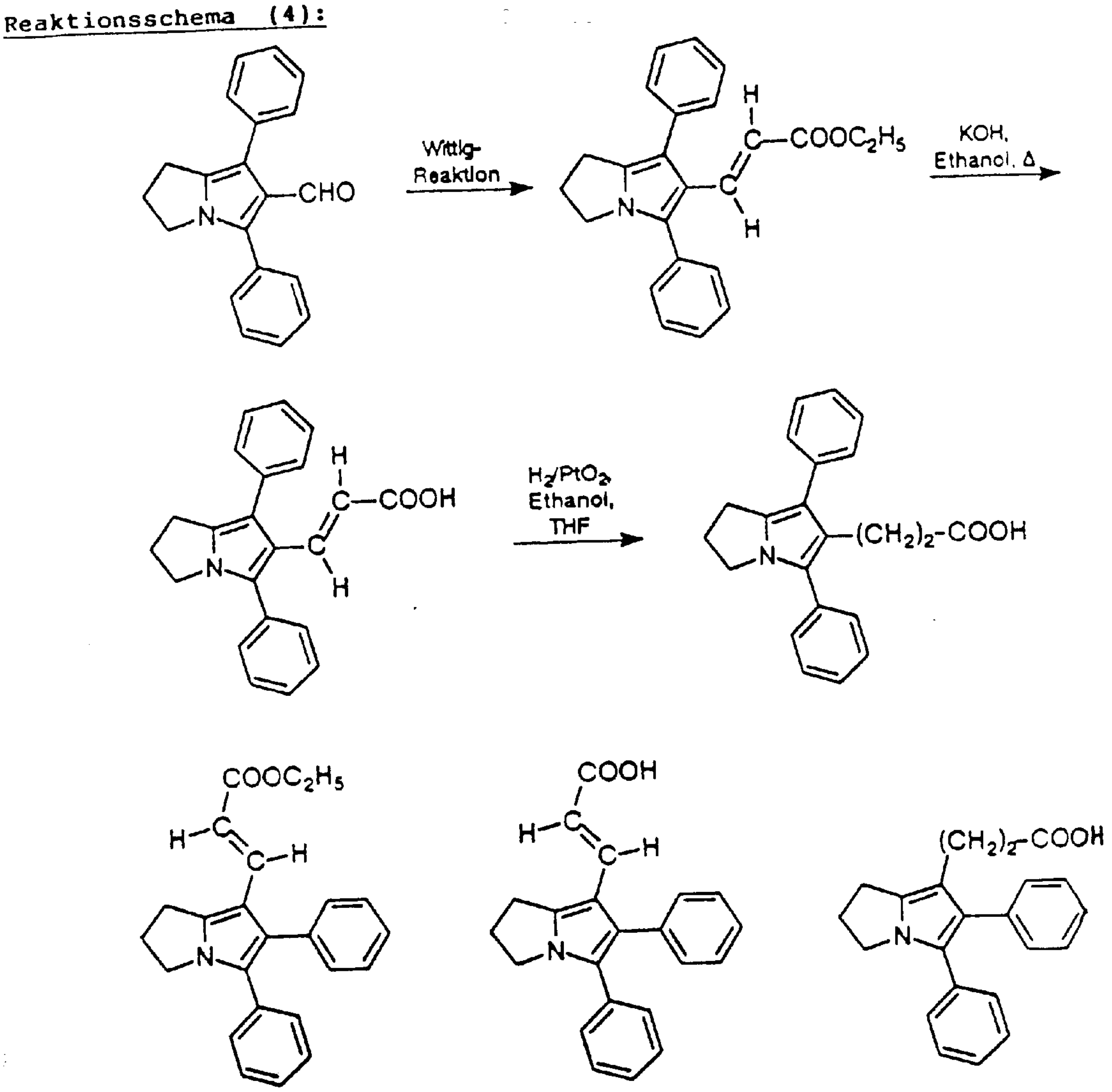


Die Herstellung der entsprechenden Buttersäure-Derivate erfolgt gemäß dem nachfolgenden Reaktionsschema (5). Dabei werden zunächst durch Friedel-Crafts-Acylierung mit Bernsteinsäureanhydrid (E. Berliner, Org. Reakt. 1949, 5, 229 - 289) die 4-Oxobuttersäuren hergestellt, die dann nach der Huang-Minlon-Variante der Wolff-Kishner-Reduktion in Diethylenglykol mit Hydrazin/KOH reduziert werden. Die Reaktionsbedingungen sind dem Fachmann bekannt.

Reaktionsschema (5):

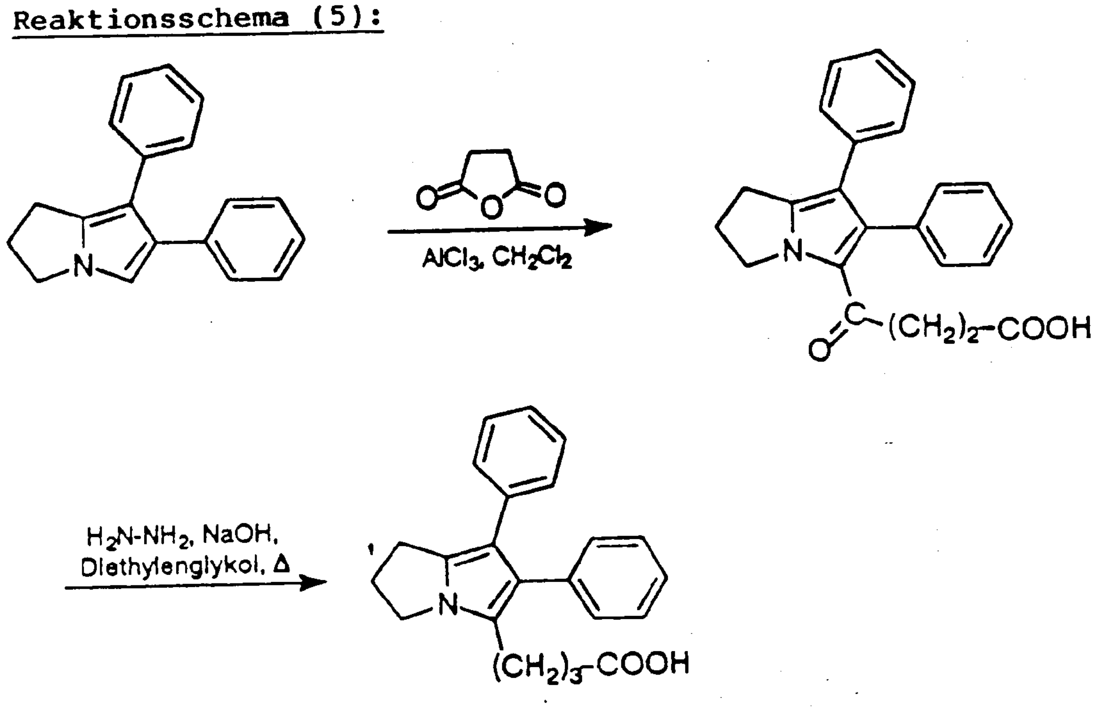


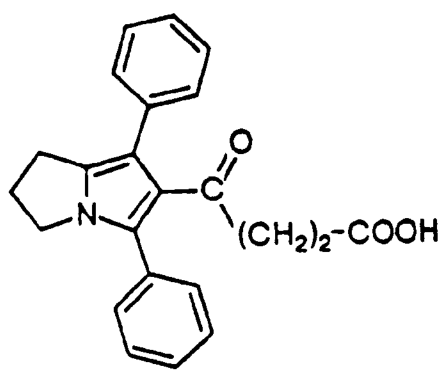

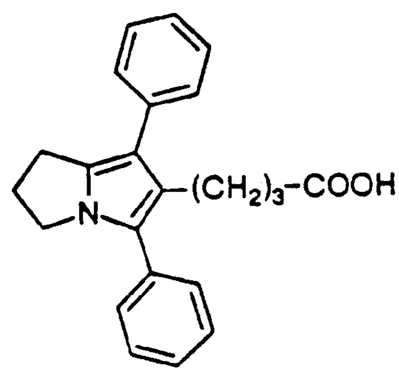

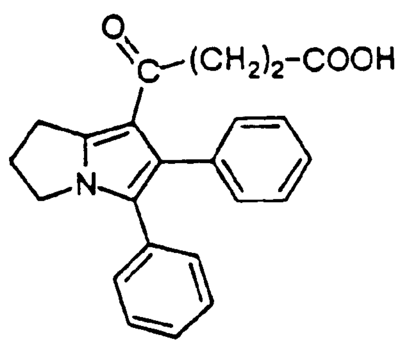

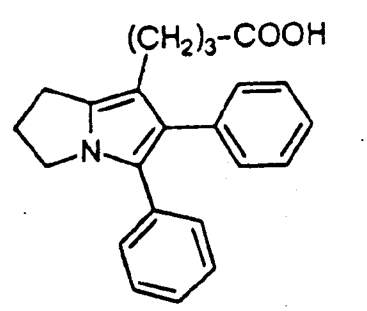

Die Valeriansäurederivate lassen sich über die 5-Oxo-valeriansäuren, die durch Friedel-Crafts-Acylierung mit Glutarsäureanhydrid aus den Pyrrolizin-Grundkörpern erhältlich sind, analog den entsprechenden Buttersäurederivaten herstellen. In entsprechender Weise werden die Capronsäure-Derivate durch Friedel-Crafts-Acylierung der Diphenylpyrrolizine mit Methyl-5-(Chlorformyl- valerat/$AlCl_3$ und Verseifung der erhaltenen 6-Oxocapronsäuremethylester-Derivate und anschließender Reduktion der Oxovaleriansäure mit Hydrazin/KOH erhalten.

Die Herstellung der an der Phenylgruppe substituierten Verbindungen erfolgt in analoger Weise. Die Hydroxysubstituierten Derivate werden dabei durch Ätherspaltung mit $BBr_3$ aus den entsprechenden Alkoxyde-

rivaten hergestellt (Tetrahedron, 1968, 24, 2289-2292).

Die erfindungsgemäßen Verbindungen haben sich als potente Cyclooxygenase- und/oder Lipoxygenasehemmer erwiesen. Sie sind daher bei der Behandlung von Erkrankungen des rheumatischen Formenkreises und zur Prävention von allergisch induzierten Erkrankungen brauchbar. Die erfindungsgemäßen Verbindungen stellen somit wirksame Antiphlogistica, Analgetica, Antipyretica, Antiallergica und Broncholytica dar und sind zur Thromboseprophylaxe und zur Prophylaxe des anaphylaktischen Schocks sowie zur Behandlung dermatologischer Erkrankungen, wie Psoriasis, Urtikaria, akute und chronische Exantheme allergischer und nicht-allergischer Genese brauchbar.

Die erfindungsgemäßen Verbindungen können entweder als einzelne therapeutische Wirkstoffe oder als Mischungen mit anderen therapeutischen Wirkstoffen verabreicht werden. Sie können alleine verabreicht werden, im allgemeinen werden sie jedoch in Form pharmazeutischer Mittel verabreicht, das heißt, als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wäßriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Die Wirksamkeit der erfindungsgemäßen Verbindungen läßt sich anhand der Hemmung der 5-Lipoxygenase oder der Cyclooxygenase bestimmen. Die Untersuchungen wurden folgendermaßen durchgeführt:

Testsystem zur Bestimmung der Hemmung der 5-Lipoxygenase

Als Quelle für die 5-Lipoxygenase wurden Rindergranulocyten gewählt, die wie die menschlichen Granulocyten zur Bildung von Leukotrienen befähigt sind. Durch Stimulation mit Calcium-Ionophor (siehe Biochem. Biophys. Acta 1984, 795, 499 bis 503) werden hauptsächlich $LTC_4$ (Leukotrien $C_4$) und $LTB_4$ (Leukotrien $B_4$) aus endogener Arachidonsäure gebildet. Die Isolierung der Granulocyten und die Durchführung der Enzymreaktion erfolgt nach bekannten Verfahren (siehe Biochem., Biophys. Acta 1984, 795, 499 - 503). Das mit EDTA vor Gerinnung geschützte Blut wird dazu zunächst zentrifugiert und der thrombozytenreiche Überstand wird entfernt. Nach Lyse der Erythrocyten mit Wasser werden Lymphocyten und Monocyten von den Granulocyten mit Hilfe eines Ficoll-Gradienten getrennt. Die Granulocyten werden auf eine bestimmte Zellzahl eingestellt. Die Enzymreaktion wird dann in Anwesenheit bzw. Abwesenheit der Testsubstanzen nach Zugabe von $Ca2^+$ mit Calcium-Ionophor gestartet. Die Synthese der Leukotriene wird nach 5 Minuten durch Zusatz einer Mischung von Methanol und Acetonitril, die PGB2 als internen Standard und NDGA als Antioxidans enthält, gestoppt. Die Proben werden dann in Wasser verdünnt und wie in J. Chromatogr. 1986, 378, 208 - 214 beschrieben, aufgearbeitet. Die Messung von $LTB_4$ erfolgt im Absorptionsmaximum bei 270 nm. Die Arachidonsäuremetabolyten werden bei dieser Untersuchung annähernd quantitativ wiedergefunden.

Testsystem zur Bestimmung der Hemmung der Cyclooxygenase

Bei diesem Testsystem wird die von Rinderthrombocyten nach Zusatz von Calcium-Ionophor gebildete 12-HHT-Menge 12-Hydroxyheptadecatriensäure bzw. Prostaglandin $E_2$-Menge durch UV-Detektion nach HPLC-Trennung bestimmt. Dabei werden die Thrombocyten nach Zentrifugation des Rinderblutes aus dem erhaltenen Überstand gewonnen. Die Enzymreaktion und die Isolierung der gebildeten Metaboliten erfolgt wie bei der Bestimmung der 5-Lipoxygenase-Hemmung, wobei jedoch die Inkubationszeit eine Minute betragen hat. Die Detektion von 12-HHT nach HPLC-Trennung erfolgt bei 232 nm.

Die bei der Testung der erfindungsgemäßen Verbindungen erhaltenen Ergebnisse sind in den nachfolgenden Tabellen 1 und 2 zusammengestellt. Die Testsubstanzen kamen dabei in einer Konzentration von 10 μM zur Anwendung.

Tabelle 1: Hemmung der 5-Lipoxygenase und der Cyclooxygenase durch Pyrrolyzinverbindungen

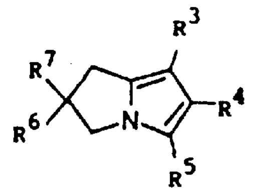

(10 µM)

| Beisp. Nr. | $R^3$ | $R^4$ | $R^5$ | $R^6$ | $R^7$ | Hemmung % Lipoxy-genase | Cyclooxy-genase |
|---|---|---|---|---|---|---|---|
| 11 | Phenyl | m-Chlorphenyl | $(CH_2)_2CO_2H$ | H | H | 89 | |
| 12 | Phenyl | p-Chlorphenyl | $(CH_2)_2CO_2H$ | H | H | 98 | |
| 51 | Phenyl | p-Tolyl | $(CH_2)_2CO_2H$ | H | H | 92 | |
| 53 | Phenyl | p-Methoxy-phenyl | $(CH_2)_2CO_2H$ | H | H | 91 | |
| 58 | Phenyl | p-Phenoxy-phenyl | $(CH_2)_2CO_2H$ | H | H | 98 | 70 |
| 68 | Phenyl | p-Fluorphenyl | $(CH_2)_2CO_2H$ | $CH_3$ | $CH_3$ | | 93 (3.3 µM) |
| | Phenyl | p-Tolyl | $(CH_2)_2CO_2H$ | $CH_3$ | $CH_3$ | | 93 (3.3 µM) |
| 75 | Phenyl | p-Phenoxy-phenyl | $(CH_2)_2CO_2H$ | $CH_3$ | $CH_3$ | 98 (3,3 µM | |

Die nachfolgenden Beispiele erläutern die Erfindung. Alle Temperaturangaben sind unkorrigiert. Die IR-Spektren wurden, soweit nicht anders angegeben, mit KBr-Preßlingen aufgenommen. Die NMR-Spektren sind, sofern nicht anders vermerkt, 90 MHz-Spektren, aufgenommen in $CDCl_3$ mit Tetramethylsilan (TMS) als internem Standard.

Die Herstellung und Charakterisierung der erfindungsgemäßen Verbindungen bzw. der teilweise für die folgenden Umsetzungen als Ausgangsprodukte verwendeten Verbindungen erfolgt analog zu:
Arch. Pharm. 312, 896 - 907 (1979)
Arch. Pharm. 319, 500 - 505 (1986)
Arch. Pharm. 321, 159 - 162 (1988)
Arch. Pharm 321, 545 - 549 (1988)
Chemiker Zeitung, 110 (1986), 7/8, 267 - 271

Allgemeine Arbeitsvorschrift zur Darstellung der 4-(Diphenyl-2,3-dihydro-1H-pyrrolizinyl)-4-oxobuttersäuren

Zur Lösung von 4 mmol Diphenyl-2,3-dihydro-1H-pyrrolizin und 4 mmol (0.40 g) Bernsteinsäureanhydrid in 16 ml absol. $CH_2Cl_2$ werden unter Eiskühlung und unter Rühren innerhalb von 5 min portionsweise 8.8 mmol (1.17 g) $AlCl_3$ zugesetzt. Anschließend rührt man 45 min bei RT. Der Ansatz wird dann in 150 ml Eiswasser gegossen. Nach Zusatz von 4 ml 8%iger $H_3PO_4$ extrahiert man dreimal mit $CHCl_3$, trocknet die organischen Phasen über $Na_2SO_4$ und destilliert das Lösungsmittel ab. Die Isolierung des Produktes erfolgt mittels SC (Kieselgel, 1.Ether, 2.Ether/THF 1+1). Die Produkffraktionen werden eingeengt, der wird Rückstand in wenig $CHCl_3$ gelöst. Nach Zusatz von n-Hexan und erneutem Einengen fällt das Produkt aus.

Allgemeine Arbeitsvorschrift zur Darstellung der 4-(Diphenyl-2,3-dihydro-1H-pyrrolizinyl)-buttersäuren

0.5 mmol 4-(Diphenyl-2,3-dihydro-1H-pyrrolizinyl)-4-oxobuttersäure werden mit 10 ml Diethylenglykol, 50 mmol (2.8 g KOH und 30 mmol (1.5 g Hydrazin Hydrat versetzt. Der Ansatz wird 1 h zum Rückfluß erhitzt

(Badtemperatur ca. 170°C). Anschließend ersetzt man den Rücklußkühler durch eine Destillationsbrücke und heizt weiter auf, bis eine Innentemperatur von ca. 210°C erreicht ist. Diese Temperatur wird dann 2 h gehalten. Nach dem Abkühlen gießt man den Ansatz in 150 ml $H_2O$, säuert mit 8%iger $H_3PO_4$ an und extrahiert dreimal mit Ether. Die Extrakte werden mit $H_2O$ gewaschen, getrocknet und eingeengt. Das Produkt wird durch SC (z.B. Kieselgel, Ether/THF 4+1, Diisopropylether, Ether und Ether/n-Hexan 4+1) isoliert und mit n-Hexan ausgefällt.

Allgemeine Arbeitsvorschrift zur Darstellung der 5-(Diphenyl-2,3-dihydro-1H-pyrrolizinyl)-5-oxovaleriansäuren

4 mmol Diphenyl-2,3-dihydro-1H-pyrrolizin werden in 16 ml absol. $CH_2Cl_2$ mit 4 mmol (0.46 g) Glutarsäureanhydrid und 8.8 mmol (1.17 g) $AlCl_3$ analog der AAV zur Darstellung der 4-(Diphenyl-2,3-dihydro-1H-pyrrolizinyl)-4-oxobuttersäuren umgesetzt. Abweichend davon wird der Ansatz unmittelbar nach dem $AlCl_3$-Zusatz in Eiswasser gegossen und weiter aufgearbeitet. Die Reinigung des Produktes erfolgt mittels Säulenchromatographie (Kieselgel/Ether).

Allgemeine Arbeitsvorschrift zur Darstellung der 5-(Diphenyl-2,3-dihydro-1H-pyrrolizinyl)-5-valeriansäuren

0.5 mmol 5-(Diphenyl-2,3-dihydro-1H-pyrrolizinyl)-5-oxovaleriansäuren werden analog der AAV zur Darstellung der 4-(Diphenyl-2,3-dihydro-1H-pyrrolizinyl)-4-oxobuttersäuren umgesetzt. Das Produkt wird durch SC (Kieselgel, Ether) isoliert und mit n-Hexan ausgefällt.

Allgemeine Arbeitsvorschrift zur Darstellung der 6-(Diphenyl-2,3-dihydro-1H-pyrrolizinyl)-6-oxocapronsäuremethylester

Zur Lösung von 10 mmol Diphenyl-2,3-dihydro-1H-pyrrolizin und 10 mmol (1.79 g) Methyl-5-(chlorformyl)-valerat in 50 ml absol. $CH_2Cl_2$ werden unter Eiskühlung und unter Rühren innerhalb 5 min portionsweise 22 mmol (2.93 g) $AlCl_3$ zugesetzt. Unmittelbar danach wird der Ansatz in 150 ml eisgekühlte 10%ige NaCl-Lösung gegossen. Nach Zusatz von 4 ml 8%iger $H_3PO_4$ extrahiert man dreimal mit Ether, trocknet die organischen Phasen über $Na_2SO_4$ und destiliert das Lösungsmittel ab. Die Isolierung des Produktes erfolgt mittels SC (z.B. Kieselgel, 1. n-Hexan/Ether 3+2, 2. n-Hexan/Ether 1+4 oder. 1. Toluol. 2. Hexan/Ether 1+4). Die Eluate werden zu- nächst auf ca. 100 ml eingeengt, zweimal mit 0.05n NaOH und zweimal mit 10%iger NaCl-Lösung gewaschen und schließlich über $Na_2SO_4$ getrocknet. Die Produkte fallen beim Einengen der Lösung aus oder kristallisieren nach Abdestillieren des Lösungsmittels.

Allgemeine Arbeitsvorschrift zur Darstellung der 6-(Diphenyl-2,3-dihydro-1H-pyrrolizinyl)-6-capronsäuren

Die Lösung von 1 mmol 6-(Diphenyl-2,3-dihydro-1H-pyrrolizinyl)-6-oxocapronsäuremethylester in 10 ml Ethanol wird zum Sieden erhitzt. Hierauf tropft man 5 ml 10%ige wäßrige KOH, die zuvor durch Kochen entgast worden sind, zu und erhitzt noch 15 min unter Rückfluß. Nach dem Abkühlen gießt man den Ansatz in 100 ml 5%ige NaCl, säuert mit 8%iger $H_3PO_4$ an und extrahiert dreimal mit Ether. Die organischen Phasen werden über $Na_2SO_4$ getrocknet und eingeengt. Der Rückstand wird mit 50 mmol (2.8 g) KOH und 30 mmol (1.5 g) Hydrazin-Hydrat in 10 ml Diethylenglykol analog der AAV zur Darstellung der 4-(Diphenyl-2,3-dihydro-1H-pyrrodizinyl)-buttersäuren umgesetzt und durch Säulenchromatographie an Kieselgel gereinigt.

Allgemeine Arbeitsvorschrift zur Darstellung der 2-(Diphenyl-2,3-dihydro-1H-pyrrolizinyl)-propionsäureethylester

5 mmol Diphenyl-2,3-dihydro-1H-pyrrolizin, gelöst in 4 ml absol. Toluol, wer- den mit 7,5 mmol (0.96 g) 2-Diazopropionsäureethylester, gelöst in 2 ml absol. Toluol, analog der AAV zu Darstellung der Diphenyl-2,3-dihydro-1H-pyrrodizinylessigsäureethyester umgesetzt. Die Reaktionsdauer beträgt jedoch 2 h. Die Reinigung erfolgt durch SC ($Al_2O_3$, n-Hexan/Ether 9+1). Die Produkte werden mit Ethanol ausgefällt.

Allgemeine Arbeitsvorschrift zur Verseifung der 2-(Diphenyl-2,3-dihydro-1H-pyrrolizinyl)-propionsäureethylester

1.5 mmol Propionsäureethylester gelöst in 12 ml Ethanol, werden mit 8 ml 10%iger wäßriger KOH analog der AAV zu Verseifung der Diphenyl-2,3-dihydro-1H-pyrrolizinylessigsäureethylester umgesetzt. Die Verseifungsdauer beträgt bei 15 min, bis 60 min. Die Reinigung erfolgt mittels SC (Kieselgel, 1. n-Hexan/Ether 1+1, 2. Ether). Nach Einengen der Eluate werden die Produkte mit n-Hexan ausgefällt.

Referenzbeispiel 1

5-(1-(5,6-Diphenyl-2,3-dihydro-1H-pyrrolizin-7-yl)-ethyl)-2,2-dimethyl-1,3-dioxan-4,6-dion (59a)

Die Lösung von 4 mmol (1.04 g) 5,6-Diphenyl-2,3-dihydro-1H-pyrrolizin 4 mmol (0.58 g) 2,2-Dimethyl-1,3-dioxan-4,6-dion (Meldrum's Säure) und 8 mmol (0.35 g) frisch destilliertem Acetaldehyd in 40 ml Acetonitril wird 24 h bei 30°C stehengelassen. Nach Abkühlen im Eisbad saugt man das ausgefallene Produkt ab.
Ausbeute: 0.44 (26 %)
Schmp.: 169 °C (unter Zers.)
IR: $\nu_{max}$ = 1790(C=O), 1755(C=O), 1605 (C=C) $cm^{-1}$

Referenzbeispiel 2

3-(5,6-Diphenyl-2,3-dihydro-1H-pyrrolizin-7-yl)-buttersäureethylester (60a)

1 mmol der Verbindung des Referenzbeispiels 1 wird in einer Mischung von 5 ml absol. Pyridin und 1.0 ml absol. Ethanol gelöst. Nach Zusatz von etwas Kupferpulver wird 3 h zum Rückfluß erhitzt Das Kupferpulver wird abgesaugt und das Lösungsmittel im Vakuum abdestilliert Aus dem Rückstand wird das Produkt mittels SC (Kieselgel, n-Hexan/$CH_2Cl_2$ 1+2) isoliert.
Ausbeute: 95mg (25%)
IR: $\nu_{max}$ = 1735 (C=O), 1605 (C=C) $cm^{-1}$

AAV (Allgemeine Arbeitsvorschrift) zur Darstellung der 6-Aryl-7-phenyl-2,3-dihydro-1H-pyrrolizine bzw. der 6-Phenyl-7-aryl-2,3-dihydro-1H-pyrrolizine

20 mmol aromatensubstituiertes * bzw. unsubstituiertes α-Bromacetophenon, gelöst in 25 ml $CH_2Cl_2$ werden mit der Lösung von 20 mmol unsubstituiertem bzw. aromatensubstituiertem ** 2-Benzyl-Δ1-pyrrolin in 50 ml Ethanol versetzt und 24 h bei RT gerührt. Anschließend gibt man 20 ml gesättigte wäßrige $NaHCO_3$-Lösung zu und rührt weitere 24 h bei RT. Der Ansatz wird in 500 ml 5%ige NaCl-Lösung gegossen und dreimal mit je

* α-Brom-3-chloracetophenon, α-Brom-3,4-dimethoxyacetophenon, α-Brom-3,4-dichloracetophenon und α-Brom-4-phenoxyacetophenon sind nicht käuflich erwerbbar. Sie werden wie folgt hergestellt:
Zur Lösung von 20 mmol 3-Chloracetophenon, 3,4-Dimethoxyacetophenon, 3,4-Dichloracetophenon bzw. 4-Phenoxyacetophenon in 15 ml $CH_2Cl_2$ und 10 ml Dioxan wird die Lösung von 20 mmol (3.2 g) Brom in 10 ml $CH_2Cl_2$ langsam zugetropft. Anschließend versetzt man mit 50 ml $CH_2Cl_2$ und wäscht zur Entfernung von HBr zweimal vorsichtig mit 5%-iger $NaHCO_3$-Lösung. Die organische Phase wird über $Na_2SO_4$ getrocknet und eingeengt. Der Rückstand wird direkt wie oben beschrieben mit 20 mmol 2-Benzyl-Δ1-pyrrolin umgesetzt.
** 2-(4-Chlorbenzyl)-Δ1-pyrrolin
Darstellung analog 2-Benzyl-Δ1-pyrrolin (J.Amer.Chem.Soc.1932, 54 3971-3976)
** 2-(4-Methylbenzyl)-Δ1-pyrrolin
Aus 0.15 mol (3.6 g) Mg und 0.15 mol (21.1g) 4-Methylbenzylchlorid wird in 150 mol absol. Ether ein Grignard-Reagens hergestellt. Nach Zutropfen von 0.15 mol (15.5 g) 4-Chlorbutyronitril, gelöst in 100 ml absol. Ether, wird 2 h zum Rückfluß erhitzt. Anschließend destilliert man den Ether ab und gibt 200 ml absol. Xylol zu. Nach weiteren 2 h Rückflußkochen wird der Ansatz unter Eiskühlung mit 100 ml $H_2O$ versetzt und mit verd. $H_3PO_4$ angesäuert. Die wäßrige Phase wird unter Eiskühlung mit konz. $NH_3$ alkalisiert, der entstehende Niederschlag abgesaugt. Das Filtrat wird dreimal mit 50 ml $CH_2Cl_2$ extrahiert, der Niederschlag mit 100 ml $CH_2Cl_2$ gewaschen. Die $CH_2Cl_2$-Lösungen werden vereinigt, über $Na_2SO_4$ getrocknet und eingeent. Aus dem verbleibenden Rückstand isoliert man das Produkt durch Destillation (Siedepunkt 117°C bei 0.1 Torr).
Ausbeute: 3.7 g (14 %)
$C_{12}H_{15}N$ (173.3)
IR: $\nu_{max}$ = 1640 (C=N), 1605 (C=C) $cm^{-1}$
** 2-(4-Methoxybenzyl)-Δ1-pyrrolin
Aus 3.0 mol (72.9 g) Mg und 0.15 mol (23.5 g) 4-Methoxybenzylchlorid wird in 500 ml absol. Ether 4-Methoxybenzylmagnesiumchlorid hergestellt. Dieses wird mit 0.15 mol (15.5 g) 4-Chlorbutyronitril weiter umgesetzt und durch Destillation gereinigt (Siedepunkt 142 °C bei 0.1 Torr).
Ausbeute: 2.4 g (8 %)
$C_{12}H_{15}NO$ (189.3)
IR: $\nu_{max}$ = 1640 (C=N), 1610 (C=C) $cm^{-1}$

100 ml Ether/$CH_2Cl_2$3+1 extrahiert. Die organishcen Phasen werden über $Na_2SO_4$ getrocknet und wie unten angegeben aufgearbeitet.

Allgemeine Arbeitsvorschrift zur Darstellung der 3-(6-Chlorphenyl- und 6-Nitrophenyl-7-phenyl-2,3-dihydro-1H-pyrrolizin-5-yl)propionsäuremethylester

4 mmol des 6-Chlor- bzw. 6-Nitrophenyl-7-phenyl-2,3-dihydro-1H-pyrrolizins und 6 mmol (0.52 g) Acrylsäuremethylester werden in 16 ml absol. Dichlorethan gelöst. Nach Zusatz von 0.24 ml $BF_3$-Ethylether-Komplex wird 1 h bei RT gerührt. wobei nach jeweils 15 min nochmals die gleichen Mengen an Acrylsäuremethylester und an $BF_3$-Ethylether-Komplex zugegeben werden. Anschließend gießt man den Ansatz in 100 ml 10%-ige NaCl-Lösung und extrahiert zweimal mit Ether/$CH_2Cl_2$ 3+1. Die organischen Phasen werden über $Na_2SO_4$ getrocknet und eingeengt. Der Rückstand wird wie unten angegeben aufgearbeitet.

Allgemeine Arbeitsvorschrift zur Verseifung der 3-(6-Chlorphenyl- und 6-Nitrophenyl-7-phenyl-2,3-dihydro-1H-pyrrolizin-6-yl)-propionsäuremethylester

Die Lösung von 1 mmol des entsprechenden Pyrrolizinylpropionsäuremethylesters in 10 ml Ethanol wird zum Sieden erhitzt. Hierauf tropft man 5 ml 10%ige wäßrige KOH zu, die zuvor durch Kochen entgast worden ist, und erhitzt noch 5 min unter Rückfluß. Nach dem Abkühlen gießt man den Ansatz in 100 ml 5%-ige NaCl-Lösung, säuert mit 8%-iger $H_3PO_4$ an und extrahiert dreimal mit Ether/$CH_2Cl_2$ 3+1. Die organischen Phasen werden über $Na_2SO_4$ getrocknet, eingeengt und wie angegeben aufgearbeitet.

Allgemeine Arbeitsvorschrift für die Vilsmeier-Formylierung aromatensubstituierter 6,7-Diphenyl-2,3-dihydro-1H-pyrrolizine

6 mmol des entsprechenden Pyrrolizins, gelöst in 6 ml absol. Benzol, werden mit 18 mmol (1.32 g) absol. DMF und 6 mmol (0.92 g) $POCl_3$ analog der AAV für die Vilsmeier-Formylierung der Diphenyl-2,3-dihydro-1H-pyrrolizine umgesetzt.

Allgemeine Arbeitsvorschrift zur Darstellung aromatensubstituierter 6,7-Diphenyl-2,3-dihydro-1H-pyrrolizinylacrylsäureethylester

2.5 mmol des entsprechenden Carbaldehyds, gelöst in 5 ml absol. $CH_2Cl_2$, werden mit der Lösung von 2.5 mmol (1.08 g) Ethoxycarbonylmethyltriphenylphosphoniumbromid in 4 ml absol. Ethanol und einer aus 7.5 mmol (0.17 g) Natrium und 3 ml absol. Ethanol bereiteten Lösung von Na-Ethanolat analog der AAV zur Darstellung der Diphenyl-2,3-dihydro-1H-pynrolizinylacrylsäure ethylester umgesetzt.

Allgemeine Arbeitsvorschrift zur Verseifung aromatensubstituierter 3-(6,7-Diphenyl-2,3-dihydro-1H-pyrrolizin-5-yl)-acrylsäureethylester

1.2 mmol des entsprechenden Pyrrolizinylacrylsäureethylesters, gelöst in 50 ml Ethanol, werden mit 10 ml wäßrige KOH analog der AAV zur Verseifung der 3-(Diphenyl-2,3-dihydro-1H-pyrrolizin-5-yl)-acrylsäureethylester umgesetzt.

Allgemeine Arbeitsvorschrift zur Hydrierung aromatensubstituierter 3-(6,7-Diphenyl-2,3-dihydro-1H-pyrrolizin-5-yl)-acrylsäuren

0.9 mmol der entsprechenden Pyrrolizinylacrylsäure werden in 30 ml absol. THF gelöst. Nach Zusatz von 10 ml absol. Ethanol und einer Spatelspitze $PtO_2$(alternativ kann auch Palladium verwendet werden) hydriert man im Autoklaven ca. 4 h bei 15 bar. Dabei wird mehrmals eine Spatelspitze frisches $PtO_2$ (bzw. Palladium) zugesetzt. Nach vollständiger Umsetzung (DC: Kieselgel, THF) wird der Katalysator abgesaugt, das Lösungsmittel abdestilliert und das Produkt isoliert.

Allgemeine Arbeitsvorschrift zur Spaltung der Arylmethylether

0.5 mmol der entsprechenden Methoxyverbindung, gelöst in 5 ml absol. $CH_2Cl_2$, werden bei -80°C zur Lösung von 0.20 ml $BBr_3$ in 3 ml absol. $CH_2Cl_2$ zugetroft. Man läßt die Mischung während ca. 8 h auf RT erwärmen. Nach Zusatz von 30 ml $H_2O$ wird dreimal mit Ether extrahiert. Die organischen Phasen werden zweimal mit

gesättigter NaCl-Lösung gewaschen, über $Na_2SO_4$ getrocknet und aufgearbeitet.

Die erhaltenen Verbindungen und physikalische Daten dieser Verbindungen sind in nachfolgenden Tabellen 2 bis 10 zusammengestellt.

Bei den Verbindungen in den Tabellen 2,5,6 und der Verbindung 61 in Tabelle 10 handelt es sich um Ausgangsverbindungen.

Tabelle 2

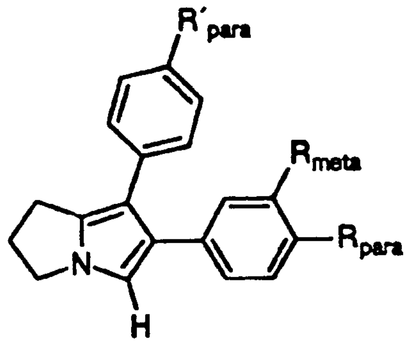

| Beisp. Nr. | $R_{meta}$ | $R_{para}$ | $R'_{para}$ | Schmp. °C | IR(C=C) |
|---|---|---|---|---|---|
| 1 | Cl | H | H | | 1600 |
| 2[1)] | H | Cl | H | | |
| 3 | $NO_2$ | H | H | 117 | 1605 |
| 4 | H | $NO_2$ | H | 132 | 1595 |
| 5 | H | H | Cl | 156 | 1605 |

1) Chemiker-Zeitung 1986, 110, 267-271

Tabelle 3

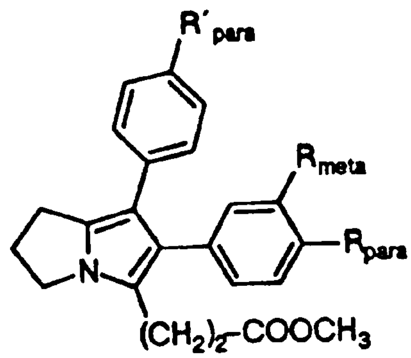

| Beisp. Nr. | $R_{meta}$ | $R_{para}$ | $R'_{para}$ | Schmp. °C | IR(C=O) |
|---|---|---|---|---|---|
| 6 | Cl | H | H | 99 | 1735 |
| 7 | H | Cl | H | 98 | 1730 |
| 8 | $NO_2$ | H | H | | 1740 |
| 9 | H | $NO_2$ | H | | 1740 |
| 10 | H | H | Cl | 111 | 1730 |

Tabelle 4

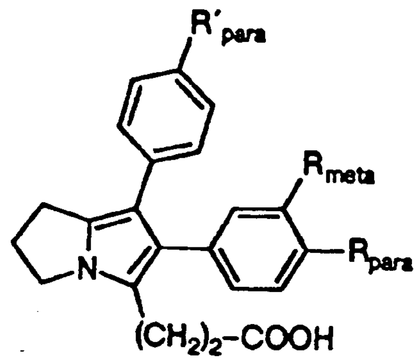

| Beisp. Nr. | $R_{meta}$ | $R_{para}$ | $R'_{para}$ | Schmp. °C | IR(C=O) |
|---|---|---|---|---|---|
| 11 | Cl | H | H | 99 | 1735 |
| 12 | H | Cl | H | 98 | 1730 |
| 13 | $NO_2$ | H | H | | 1740 |
| 14 | H | $NO_2$ | H | | 1740 |
| 15 | H | H | Cl | 111 | 1730 |

Tabelle 5

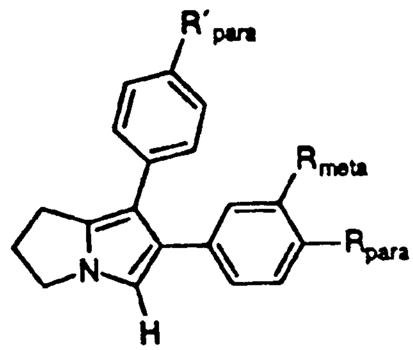

| Beisp. Nr. | $R_{meta}$ | $R_{para}$ | $R'_{para}$ | Schmp. °C | OR(C=C) |
|---|---|---|---|---|---|
| 16 | $CH_3$ | H | H | | 1605 |
| 17 | H | $CH_3$ | H | | 1605 |
| 18 | $OCH_3$ | H | H | | 1605 |
| 19[1)] | H | $OCH_3$ | H | | |
| 20[1)] | $OCH_3$ | $OCH_3$ | H | | |
| 21[2)] | H | $O-C_6H_5$ | H | | |
| 22 | Cl | Cl | H | 119 | 1605 |
| 23 | H | H | $CH_3$ | 104 | 1610 |
| 24 | H | H | $OCH_3$ | | 1605 |

1) Chemiker-Zeitung 1986 110, 267-271

2) Das Produkt ist nicht rein; es wird ohne weitere Reinigung umgesetzt.

Tabelle 6

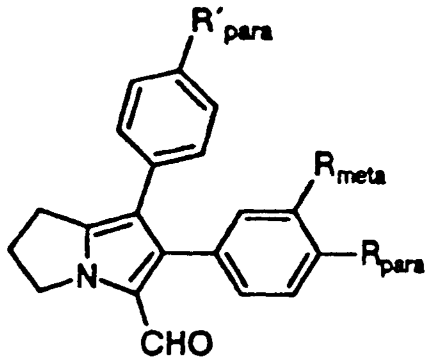

| Beisp. Nr. | $R_{meta}$ | $R_{para}$ | $R'_{para}$ | Schmp. °C | IR(C=O |
|---|---|---|---|---|---|
| 26 | $CH_3$ | H | H | 128 | 1645 |
| 27 | H | $CH_3$ | H | 142 | 1645 |
| 28 | $OCH_3$ | H | H | 117 | 1635 |
| 29[1)] | H | $OCH_3$ | H | | |
| 30[1)] | $OCH_3$ | $OCH_3$ | H | | |
| 31 | H | $O-C_6H_5$ | H | 135 | 1635 |
| 32 | Cl | Cl | H | 162 | 1640 |
| 33 | H | H | $CH_3$ | 102 | 1645 |

1) Chemiker-Zeitung 1986 110, 267-271

Tabelle 7

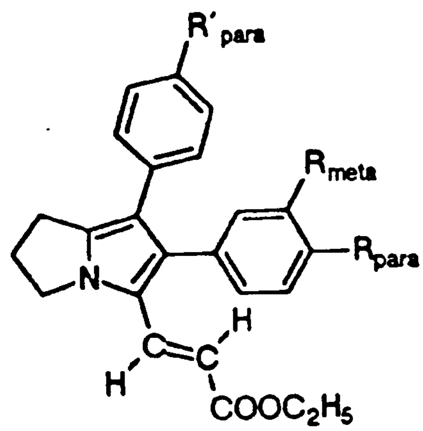

| Beisp. Nr. | $R_{meta}$ | $R_{para}$ | $R'_{para}$ | Schmp. °C | IR(C=O) |
|---|---|---|---|---|---|
| 34 | $CH_3$ | H | H | 142 | 1710 |
| 35 | H | $CH_3$ | H | 158 | 1710 |
| 36 | $OCH_3$ | H | H | 119 | 1710 |
| 37 | H | $OCH_3$ | H | 164 | 1705 |
| 38 | $OCH_3$ | $OCH_3$ | H | 133 | 1705 |
| 39 | H | $O-C_6H_5$ | H | 173 | 1705 |
| 40 | Cl | Cl | H | 122 | 1715 |
| 41 | H | H | $CH_3$ | 222 | 1705 |

Tabelle 8

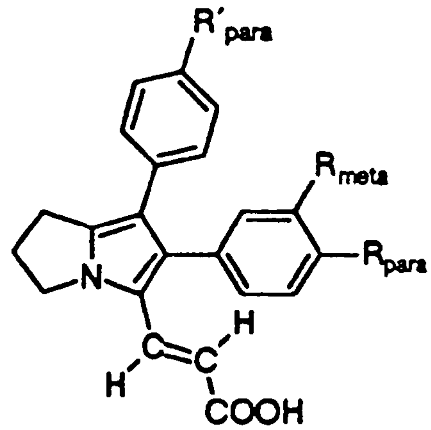

| Beisp. Nr. | $R_{meta}$ | $R_{para}$ | $R'_{para}$ | Schmp. °C | IR(C=O) |
|---|---|---|---|---|---|
| 42 | $CH_3$ | H | H | 216 | 1660 |
| 43 | H | $CH_3$ | H | 221 | 1680 |
| 44 | $OCH_3$ | H | H | 205 | 1670 |
| 45 | H | $OCH_3$ | H | 216 | 1675 |
| 46 | $OCH_3$ | $OCH_3$ | H | 216 | 1660 |
| 47 | H | $O-C_6H_5$ | H | 221 | 1660 |
| 48 | Cl | Cl | H | 229 | 1660 |
| 49 | H | H | $CH_3$ | 118 | 1665 |

Tabelle 9

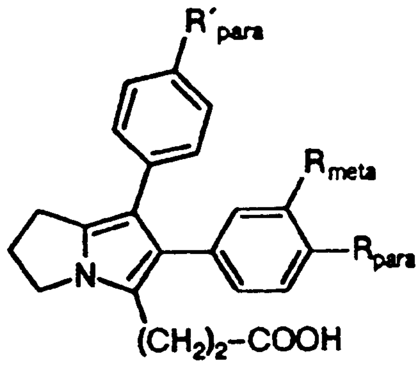

| Beisp. Nr. | $R_{meta}$ | $R_{para}$ | $R'_{para}$ | Schmp. °C | IR(C=O) |
|---|---|---|---|---|---|
| 50 | $CH_3$ | H | H | 125 | 1710 |
| 51 | H | $CH_3$ | H | 122 | 1710 |
| 52 | $OCH_3$ | H | H | 111 | 1710 |
| 53 | H | $OCH_3$ | H | 73 | 1710 |
| 54 | $OCH_3$ | $OCH_3$ | H | 152 | 1725 |
| 55 | OH | H | H | 87 | 1710 |
| 56 | H | OH | H | 187 | 1700 |
| 57 | OH | OH | H | 76 | 1710 |
| 58 | H | $O-C_6H_5$ | H | 178 | 1705 |
| 59 | Cl | Cl | H | 144 | 1710 |
| 60 | H | H | $CH_3$ | 182 | 1705 |

Tabelle 10

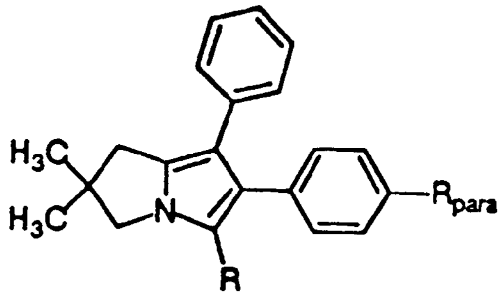

| Beisp. Nr. | R | $R_{para}$ | Schmp. °C | IR(C=O) |
|---|---|---|---|---|
| 61 | H | Cl | 118 | 1605(C=C) |
| 62 | $(CH_2)_2COOCH_3$ | Cl | 113 | 1735 |
| 63 | $(CH_2)_2COOH$ | Cl | 207 | 1710 |

Beispiel 64

2,2-Dimethyl-6-(4-fluorphenyl)-7-phenyl-2,3-dihydro-1H-pyrrolizin (1v)

20 mmol α-Chlor-4-fluoracetophenon, gelöst in 25 ml $CH_2Cl_2$, werden mit der Lösung von 20 mmol 2-Benzyl-4,4-dimethyl-Δ1-pyrrolin 25 ml Ethanol versetzt und 24 h bei 70°C Badtemperatur gerührt. Anschließend gibt man 20 ml gesättigte wäßrige $NaHCO_3$-Lösung zu und rührt weitere 24 h bei gleicher Temperatur. Der Ansatz wird in 500 ml 5%-ige NaCl-Lösung gegossen und dreimal mit je 100 ml Ether/$CH_2Cl_2$ 3+1 extrahiert. Die organischen Phasen werden über $Na_2SO_4$ getrocknet. Das Produkt fällt nach Reinigung mittels SC ($Al_2O_3$, n-Hexan/Ether 9+1) als Öl an.

Ausbeute: 3.1 g (51 %)

$C_{21}H_{20}FN$ (305.4)

IR: $\nu_{max}$=1605(C=C)cm$^{-1}$

$^1$H-NMR: δ(ppm)= 1.28 (s, 6H, -$CH_3$), 2.78 (s, 2H, C-1), 3.71 (s, 2H, C-3), 6.64 (s, 1H, C-5), 6.75-7.60 (m, 9H, Arom.)

Beispiel 65

2.2-Dimethyl-6-(4-fluorphenyl)-7-phenyl-2,3-dihydro-1H-pyrrolizin-5yl-carbaldehyd (6v)

9 mmol 1v, gelöst in 9 ml absol. Benzol, werden mit 27 mmol (1.97 g) absol. DMF und 9 mmol (1.38 g) $POCl_3$ analog der AAV für die Vilsmeier-Formylie- rung der Diphenyl-2,3-dihydro-1H-pyrrolizine umgesetzt. Die Reinigung erfolgt durch SC (Kieselgel, n-Hexan/Ether 2+1). Das Produkt fällt beim Einengen der Eluate aus.

Ausbeute: 0.9 g (30 %)

Schmp.: 186 °C

$C_{22}H_{20}FNO$ (333.4)
IR: $\nu_{max}$ = 1645(C=O), 1610 (C=C) $cm^{-1}$
$^1H$-NMR: δ(ppm) = 1.33 (s, 6H, $-CH_3$), 2.83 (s, 2H, C-1), 4.18 (s, 2H, C-3), 6.90-7.44 (m, 9H, Arom.), 9.38 (s, 1H, -CHO)

Beispiel 66

3-(2,2-Dimethyl-6-(4-fluorphenyl)-7-phenyl-2,3-dihydro-1H-pyrrolizin-5yl)-acrylsäureethylester (34v)

2 mmol 6v, gelöst in 15 ml absol. $CH_2Cl_2$, werden mit der Lösung von 2 mmol (0.86 g) Ethoxycarbonylmethyltriphenylphosphoniumbromid in 4 ml absol. Ethanol und einer aus 6 mmol (0.14 g) Natrium und 3 ml absol. Ethanol bereiteten Lösung von Na-Ethanolat analog der AAV zur Darstellung der Diphenyl-2,3-dihydro-1H-pyrrolizinylacrylsäureethylester umgesetzt. Die Reinigung erfolgt durch SC (Kieselgel, $CH_2Cl_2$). Das Produkt bleibt nach Einengen der Eluate als Schaum zurück.
Ausbeute: 0.33g (41 %)
Schmp.: 152°C
$C_{26}H_{26}FNO_2$ (403.5)
IR: $\nu_{max}$= 1715 (C=O), 1620 (C=C) $cm^{-1}$
$^1H$-NMR: δ (ppm) = 1.27 (t, 3H, J=7 Hz, $-O-CH_2-\underline{CH_3}$), 1.33 (s, 6H, $-CH_3$), 2.86 (s, 2H, C-1), 4.00 (s, 2H, C-3), 4.19 (q. 2H, J=7 Hz, $-O-\underline{CH_2}-CH_3$), 5.90 (AB, 1H, J=16.4 Hz, =CH-CO-), 6.87-7.34 (m, 9H, Arom.), 7.48 (AB. 1H, J=16.4 Hz, Pyr-CH=)

Beispiel 67

3-(2,2-Dimethyl-6-(4-fluorphenyl)-7-phenyl-2,3-dihydro-1H-pyrrolizin-5yl)-acrylsäure (35v)

0.6 mmol 34v, gelöst in 30 ml Ethanol werden mit 6 ml 10%-iger wäßriger KOH analog der AAV zur Verseifung der Diphenyl-2,3-dihydro-1H-pyrrolizinylacrylsäureethylester umgesetzt.
Ausbeute: 0.20 g (89%)
Schmp.: 242°C

| | |
|---|---|
| $C_{24}H_{22}FNO_2$ (375.4) | Ber. C 76.8 H 5.91 N 3.7 |
| | Gef. C 76.1 H 5.81 N 3.3 |

IR: $\nu_{max}$ = 3300-2200 (OH), 1685, 1670 (C=O), 1600 (C=C) $cm^{-1}$
$^1H$-NMR($d_6$-DMSO): δ (ppm) = 1.27 (s, 6H, $-CH_3$), 2.84 (s, 2H, C-1), 4.06 (s, 2H, C-3), 5.92 (AB, 1H, J=16.4 Hz, =CH-CO-), 6.87-7.41 (m, 9H, Arom. und Pyr-CH=)

Beispiel 68

3-(2,2-Dimethyl-6-(4-fluorphenyl)-7-phenyl-2,3-dihydro-1H-pyrrolizin-5yl)-propionsäure (27v)

0.3 mmol 35v werden analog der AAV zur Hydrierung der aromatensubstituierten 3-(6,7-Diphenyl-2,3-dihydro-1H-pyrrolizin-5-yl)-acrylsäuren umgesetzt. Als Katalysator wird Palladium verwendet. Das Produkt wird mit n-Hexan ausgefällt.
Ausbeute: 0.08 g (71 %)
Schmp.: 182°C

| | |
|---|---|
| $C_{24}H_{24}FNO_2$ (377.5) | Ber. C 76.4 H 6.41 N 3.7 |
| | Gef. C 75.8 H 6.56 N 3.3 |

IR: $\nu_{max}$ = 3300-2400 (OH), 1710 (C=O), 1605 (C=C) $cm^{-1}$
$^1H$-NMR: δ (ppm) = 1.30 (s, 6H, $-CH_3$), 2.28-2.58 (m, 2H, $-CH_2-CO-$), 2.73-3.03 (m, 2H, Pyr-$CH_2$-), 2.81(s, 2H, C-1), 3.68 (s, 2H, C-3), 6.76-7.28 (m, 9H, Arom.)

Beispiel 69

2,2-Dimethyl-6-(4-phenoxyphenyl)-7-phenyl-2,3-dihydro-1H-pyrrolizin (1w)

20 mmol 2-Benzyl-4,4-dimethyl-Δ1-pyrrolin $1b_3$ werden mit 20 mmol α-Brom-4-chloracetophenon in 50 ml Ethanol analog der AAV zur Darstellung der 6-Aryl-7-phenyl-2,3-dihydro-1H-pyrrolizine umgesetzt. Die Reinigung erfolgt mittels SC ($Al_2O_3$, n-Hexan/Ether 9+1). Nach Einengen der Eluate bleibt 1w als Öl zurück.

Ausbeute: 2.7 g (36%)
$C_{27}H_{25}NO$ (379.5)
IR: $\nu_{max}$ = 1605 und 1595 (C=C) $cm^{-1}$
$^1$H-NMR: δ (ppm) = 1.28 (s, 6H, $-CH_3$), 2.78 (s, 2H, C-1), 3.71 (s, 2H, C-3), 6.65 (s, 1H, C-5), 6.78-7.57 (m, 14H, Arom.)

Beispiel 70

2,2-Dimethyl-6-(4-phenoxyphenyl)-7-phenyl-2,3-dihydro-1H-pyrrolizin-5-yl-carbaldehyd (6w)

6 mmol 1w, gelöst in 6 ml absol. Benzol, werden mit 18 mmol (1.32 g) absol. DMF und 6 mmol (0.92 g) $POCl_3$ analog der AAV für die Vilsmeier-Formylie- rung der Diphenyl-2,3-dihydro-1H-pyrrolizine umgesetzt. Die Reinigung erfolgt durch SC (Kieselgel, $CH_2Cl_2$). Das Produkt wird mit Ethanol ausgefällt.
Ausbeute: 0.94 g (38 %)
Schmp.: 139°C
$C_{28}H_{25}NO_2$ (407.5)
IR: $\nu_{max}$ = 1645 (C=O), 1605 und 1590 (C=C) $cm^{-1}$
$^1$H-NMR: δ(ppm) = 1.30 (s, 6H, $-CH_3$), 2.81 (s, 2H, C-1), 4.17 (s, 2H, C-3), 6.84-7.47 (m, 14H, Arom.), 9.40 (s, 1H, -CHO)

Beispiel 71

3-(2,2-Dimethyl-6-(4-phenoxyphenyl)-7-phenyl-2,3-dihydro-1H-pyrrolizin-5yl)-acrylsäureethylester (34w)

2 mmol 6w, gelöst in 5 ml absol. $CH_2Cl_2$, werden mit der Lösung von 2 mmol (0.86 g) Ethoxycarbonylmethyltriphenylphosphoniumbromid in 4 ml absol. Ethanol und einer aus 6 mmol (0.14 g) Natrium und 3 ml absol. Ethanol bereiteten Lösung von Na-Ethanolat analog der AAV zur Darstellung der Diphenyl-2,3-dihydro-1H-pyrrolizinyl-acrylsäureethylester umgesetzt. Die Reinigung erfolgt durch SC (Kieselgel, $CH_2Cl_2$). Das Produkt bleibt nach Einengen der Eluate als Schaum zurück.
Ausbeute: 0.33 g (35 %)
Schmp.: ab 69°C
$C_{32}H_{31}NO_3$ (477.6)
IR: $\nu_{max}$ = 1705 (C=O), 1610 (C=C) $cm^{-1}$
$^1$H-NMR: δ (ppm) = 1.28 (t, 3H, J=7 Hz, $-O-CH_2-\underline{CH_3}$), 1.33 (s, 6H, $-CH_3$), 2.86 (s, 2H, C-1), 4.00 (s, 2H C-3), 4.19 (q, 2H, J=7 Hz, $-O-\underline{CH_2}-CH_3$), 5.92 (AB, 1H, J=16 Hz, =CH-CO-), 6.83-7.44 (m, 14H, Arom.), 7.56 (AB, 1H, J=16 Hz, Pyr-CH=)

Beispiel 72

3-(2,2-Dimethyl-6-(4-phenoxyphenyl)-7-phenyl-2,3-dihydro-1H-pyrrolizine-5yl)-acrylsäure (35w)

0.6 mmol 34w, gelöst in 30 ml Ethanol, werden mit 6 ml 10%-iger wäßriger KOH analog der AAV zur Verseifung der Diphenyl-2,3-dihydro-1H-pyrrolizin-5- yl-acrylsäureethylester umgesetzt.
Ausbeute: 0.23g (85%)
Schmp.: 198°C
$C_{30}H_{27}NO_3$ (449.5) Ber. C 80.2 H 6.05 N 3.1
Gef. C 79.9 H 6.07 N 2.7
IR: $\nu_{max}$ = 3300-2200 (OH), 1675 (C=O), 1590 (C=C) $cm^{-1}$
$^1$H-NMR ($d_6$-DMSO): δ (ppm)= 1.27 (s, 6H, $-CH_3$), 2.83 (s, 2H, C-1), 4.06 (s, 2H, C-3), 5.92 (AB, 1H, J=16.2 Hz, =CH-CO-), 6.86-7.57 (m, 15H, Arom. und Pyr-CH=)

Beispiel 73

3-(2,2-Dimethyl-6-(4-phenoxyphenyl)-7-phenyl-2,3-dihydro-1H-pyrrolizin-5yl)-propionsäure (27w)

0.3 mmol 35w werden analog der AAV zur Hydrierung der aromatensubstitu- ierten 3-(6,7-Diphenyl-2,3-dihydro-1H-pyrrolizin-5-yl)-acrylsäuren umgesetzt. Als Katalysator wird Palladium verwendet. Das Produkt wird mit n-Hexan ausgefällt.

Ausbeute: 0.10g (74%)
Schmp.: 149°C
$C_{30}H_{29}NO_3$ (451.6) Ber. C 79.8 H 6.47 N 3.1
Gef. C 79.5 H 6.61 N 2.7
IR: $\nu_{max}$ = 3300-2400 (OH), 1710 (C=O), 1605 und 1595 (C=C) $cm^{-1}$
$^1$H-NMR: δ (ppm) = 1.29 (s, 6H, $-CH_3$), 2.34-2.63 (m, 2H, $-CH_2$-CO-), 2.77-3.06 (m, 2H, Pyr-$CH_2$-), 2.83 (s, 2H, C-1), 3.69 (s, 2H, C-3), 6.85-7.47 (m, 14H, Arom.)

Beispiel 74

2-(2,2-Dimethyl-6-(4-phenoxyphenyl)-7-phenyl-2,3-dihydro-1H-pyrrolizin-5-yl)-propionsäureethylester (54a)

2.5 mmol 6-(4-Phenoxyphenyl)-7-phenyl-2,3-dihydro-1H-pyrrolizin 1w, gelöst in 3 ml absol. Toluol, werden mit 4 mmol (0.51 g) 2-Diazopropionsäureethylester, gelöst in 3 ml absol. Toluol, analog der AAV zur Darstellung der Diphenyl-2,3-dihydro-1H-pyrrolizinylessigsäureethylester (s.S.201) umgesetzt. Die Isolierung erfolgt durch SC ($Al_2O_3$, n-Hexan/Ether 9+1). Das nach Einengen der Eluate zurückbleibende ölige Produkt (0.45 g) ist nich rein. Es wird ohne zusätzliche Reinigung weiter umgesetzt.

Beispiel 75

2-(2,2-Dimethyl-6-(4-phenoxyphenyl)-7-phenyl-2,3-dihydro-1H-pyrrolizin-5-yl)propionsäure (56a)

Das verunreinigte 54a (0.45 g), gelöst in 10 ml Ethanol, wird mit 5 ml 10%-iger wäßriger KOH analog der AAV zur Verseifung der Diphenyl-2,3-dihydro-1H-pyrrolizinyl-essigsäureethylester umgesetzt. Die Verseifungsdauer beträgt 90 min. Die Reinigung erfolgt mittels SC (Kieselgel, Diisopropylether).
Ausbeute: 90 mg
Schmp.: 186°C
$C_{30}H_{29}NO_3$ (451.6)
IR: $\nu_{max}$ = 3300-2400 (OH), 1710 (C=O), 1605 und 1595 (C=C) $cm^{-1}$
1H-NMR: δ (ppm)= 1.23 (s 3H, $-CH_3$), 1.32 (s, 3H, $-CH_3$), 1.48 (d, 3H, J=7 Hz, $CH_3$-CH<), 2.72, 2.91 (AB, 2H, J=15.5 Hz, C-1), 3.81 (s, 2H, C-3), 3.96 (q, 1H, J=7 Hz, -CH<), 6.81-7.50 (m, 10H, Arom.)

## Patentansprüche

**Patentansprüche für folgende Vertragsstaaten : AT, BE, CH, DE, DK, FR, GB, IT, LI, LU, NL SE,**

1. Substituierte Pyrrolizinverbindungen der allgemeinen Formel I:

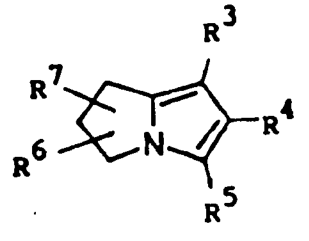

I

worin
jeweils zwei der Reste $R^3$, $R^4$ und $R^5$ unabhängig voneinander eine Arylgruppe, die gegebenenfalls durch einen oder zwei Reste substituiert ist, die ausgewählt sind unter einem Halogenatom, einer Nitro-, $C_1$-$C_4$-Alkoxy-, Hydroxy-, $C_1$-$C_4$-Alkyl- oder Phenoxygruppe
bedeuten, aber nicht gleichzeitig für eine unsubstituierte Arylgruppe stehen, und
der Dritte der Reste $R^3$, $R^4$ und $R^5$ für $-CO_2H$, -COS$C_1$-$C_4$-Alkyl oder A-X steht, wobei A eine geradkettige oder verzweigte $C_1$-$C_8$-Alkylengruppe, die gegebenenfalls durch ein Sauerstoffheteroatom oder eine Carbonylgruppe unterbrochen sein kann, oder eine $C_2$-$C_8$-Alkenylengruppe bedeutet und X für $CO_2H$, $SO_3H$, CHO, oder SH steht, und
$R^6$ und $R^7$ unabhängig voneinander für ein Wasserstoffatom oder eine $C_1$-$C_4$-Alkylgruppe stehen,

sowie deren pharmazeutisch verträgliche Salze und Ester.

2. Verbindungen nach Anspruch 1 der Formel I , worin zwei der Reste $R^3$, $R^4$ und $R^5$ für eine Phenylgruppe, die gegebenenfalls substituiert ist durch ein oder zwei Reste, die ausgewählt sind unter einem Halogenatom, insbesondere ein Fluor- oder Chloratom, einer $C_1$-$C_4$-Alkyl-, $C_1$-$C_4$-Alkoxy-, Hydroxy- Phenoxy- und Nitrogruppe, stehen und der dritte Rest für A-X steht, wobei A eine $C_1$-$C_8$-Alkylengruppe oder eine $C_2$-$C_8$-Alkenylengruppe bedeutet und X für $CO_2H$ steht.

3. Verbindungen nach Anspruch 1 der Formel:

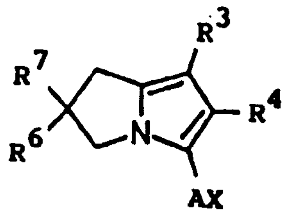

worin
$R^3$ und $R^4$ unabhängig voneinander für eine Phenylgruppe stehen, die durch ein Halogenatom substituiert sein kann, A für eine $C_1$-$C_8$-Alkylengruppe und X für $CO_2H$ steht.

4. Verbindungen nach Anspruch 3, worin $R^3$ und $R^4$ unabhängig voneinander für eine Phenylgruppe oder p-Chlorphenylgruppe stehen.

5. Verbindungen nach Anspruch 4, worin $R^6$ und $R^7$ für eine $C_1$-$C_4$-Alkylgruppe und insbesondere für eine Methylgruppe stehen.

6. Pharmazeutisches Mittel, enthaltend wenigstens eine Verbindung nach einem der Ansprüche 1 bis 5, gegebenenfalls zusammen mit üblichen pharmazeutisch verträglichen Hilfsstoffen und/oder Zusatzstoffen.

7. Verfahren zur Herstellung der Verbindungen der Ansprüche 1 bis 5 der Formel I
**dadurch gekennzeichnet**, daß man
A) eine Verbindung der allgemeinen Formel II:

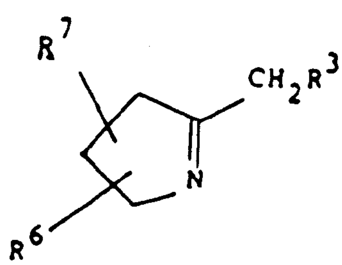

(II)

in der $R^6$ und $R^7$ unabhängig voneinander für ein Wasserstoffatom oder eine $C_1$ - $C_4$-Alkylgruppe stehen,
mit einer Verbindung der allgemeinen Formel III:

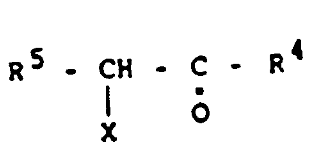

(III)

umsetzt,
worin X für Cl oder Br steht und wobei in diesen Formeln zwei der Reste $R^3$, $R^4$ und $R^5$ unabhängig voneinander die in Anspruch 1 angegebenen Bedeutungen besitzen, und der Dritte der Reste $R^3$, $R^4$

und $R^5$ für ein Wasserstoffatom steht, wobei man eine Verbindung der allgemeinen Formel IV erhält:

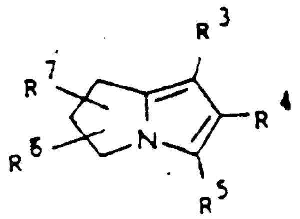

(IV)

worin die Reste $R^3$ bis $R^7$ die genannten Bedeutungen besitzen, und

B) in die so erhaltenen Verbindungen eine Hydroxymethylgruppe, Essigestergruppe, Ameisensäurethioestergruppe oder eine Acylgruppe einführt und diese gewünschtenfalls durch Reduktion, Esterspaltung, Wittig-Reaktion, Hydrierung oder durch eine Kombination dieser Maßnahmen in die gewünschte Bedeutung der Reste $R^3$, $R^4$ oder $R^5$ überführt.

8. Verwendung wenigstens einer Verbindung gemäß einem der Ansprüche 1 bis 5 zur Herstellung eines pharmazeutischen Mittels zur Prävention von allergisch induzierten Erkrankungen und zur Behandlung von Erkrankungen des rheumatischen Formenkreises.

9. Verfahren zur Herstellung des pharmazeutischen Mittels nach Anspruch 6, dadurch gekennzeichnet, daß man wenigstens eine Verbindung nach einem der Ansprüche 1 bis 5 in einer zur Verabreichung geeigneten Form bereitstellt, gegebenenfalls in üblichen pharmazeutisch verträglichen Hilfsstoffen und/oder Zusatzstoffen.

**Patentansprüche für folgenden Vertragsstaat : ES**

1. Verfahren zur Herstellung substituierter Pyrrolizinverbindungen der allgemeinen Formel I:

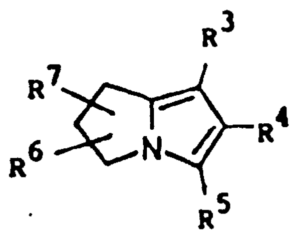

I

worin

jeweils zwei der Reste $R^3$, $R^4$ und $R^5$ unabhängig voneinander eine Arylgruppe, die gegebenenfalls durch einen oder zwei Reste substituiert ist, die ausgewählt sind unter einem Halogenatom, einer Nitro-, $C_1$-$C_4$-Alkoxy-, Hydroxy-, $C_1$-$C_4$-Alkyl- oder Phenoxygruppe

bedeuten, aber nicht gleichzeitig für eine unsubstituierte Arylgruppe stehen, und

der Dritte der Reste $R^3$, $R^4$ und $R^5$ für $-CO_2H$, $-COSC_1$-$C_4$-Alkyl oder A-X steht, wobei A eine geradkettige oder verzweigte $C_1$-$C_8$-Alkylengruppe, die gegebenenfalls durch ein Sauerstoffheteroatom oder eine Carbonylgruppe unterbrochen sein kann, oder eine $C_2$-$C_8$-Alkenylengruppe bedeutet und X für $CO_2H$, $SO_3H$, CHO, oder SH steht, und

$R^6$ und $R^7$ unabhängig voneinander für ein Wasserstoffatom oder eine $C_1$-$C_4$-Alkylgruppe stehen,

sowie der pharmazeutisch verträglichen Salze und Ester davon,

**dadurch gekennzeichnet**, daß man

A) eine Verbindung der allgemeinen Formel II:

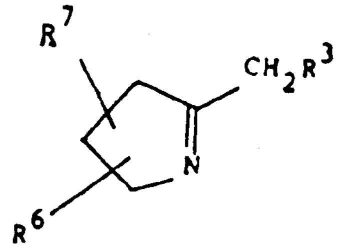

(II)

in der $R^6$ und $R^7$ unabhängig voneinander für ein Wasserstoffatom oder eine $C_1$ - $C_4$-Alkylgruppe stehen,
mit einer Verbindung der allgemeinen Formel III:

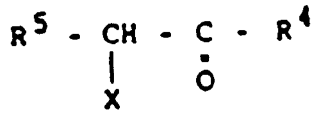

(III)

umsetzt,
worin X für Cl oder Br steht und wobei in diesen Formeln zwei der Reste $R^3$, $R^4$ und $R^5$ unabhängig voneinander die oben angegebenen Bedeutungen besitzen, und der Dritte der Reste $R^3$, $R^4$ und $R^5$ für ein Wasserstoffatom steht, wobei man eine Verbindung der allgemeinen Formel IV erhält:

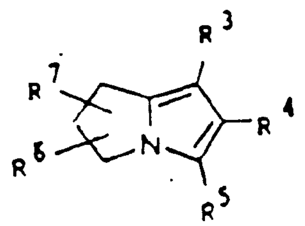

(IV)

worin die Reste $R^3$ bis $R^7$ die genannten Bedeutungen besitzen, und

B) in die so erhaltenen Verbindungen eine Hydroxymethylgruppe, Essigestergruppe, Ameisensäurethioestergruppe oder eine Acylgruppe einführt und diese gewünschtenfalls durch Reduktion, Esterspaltung, Wittig-Reaktion, Hydrierung oder durch eine Kombination dieser Maßnahmen in die gewünschte Bedeutung der Reste $R^3$, $R^4$ oder $R^5$ überführt.

2. Verfahren nach Anspruch 1, worin in Formel I zwei der Reste $R^3$, $R^4$ und $R^5$ für eine Phenylgruppe, die gegebenenfalls substituiert ist durch ein oder zwei Reste, die ausgewählt sind unter einem Halogenatom, insbesondere ein Fluor- oder Chloratom, einer $C_1$-$C_4$-Alkyl-, $C_1$-$C_4$-Alkoxy-, Hydroxy- Phenoxy- und Nitrogruppe, stehen und der dritte Rest für A-X steht, wobei A eine $C_1$-$C_8$-Alkylengruppe oder eine $C_2$-$C_8$-Alkenylengruppe bedeutet und X für $CO_2H$ steht.

3. Verfahren nach Anspruch 1, wobei man Verbindungen der Formel:

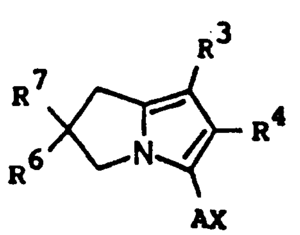

erhält, worin $R^3$ und $R^4$ unabhängig voneinander für eine Phenylgruppe stehen, die durch ein Halogenatom substituiert sein kann, A für eine $C_1$-$C_8$-Alkylengruppe und X für $CO_2H$ steht.

4. Verfahren nach Anspruch 3, wobei $R^3$ und $R^4$ unabhängig voneinander für eine Phenylgruppe oder p-Chlorphenylgruppe stehen.

5. Verfahren nach Anspruch 4, wobei $R^6$ und $R^7$ für eine $C_1$-$C_4$-Alkylgruppe und insbesondere für eine Methylgruppe stehen.

6. Verfahren zur Herstellung eines pharmazeutischen Mittels, dadurch gekennzeichnet, daß man wenigstens eine Verbindung wie in einem der Ansprüche 1 bis 5 definiert in einer zur Verabreichung geeigneten Form bereitstellt, gegebenenfalls in üblichen pharmazeutisch verträglichen Hilfsstoffen und/oder Zusatzstoffen.

**Patentansprüche für folgenden Vertragsstaat : GR**

1. Substituierte Pyrrolizinverbindungen der allgemeinen Formel I:

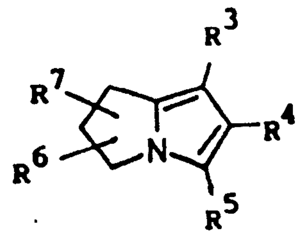

I

worin
jeweils zwei der Reste $R^3$, $R^4$ und $R^5$ unabhängig voneinander eine Arylgruppe, die gegebenenfalls durch einen oder zwei Reste substituiert ist, die ausgewählt sind unter einem Halogenatom, einer Nitro-, $C_1$-$C_4$-Alkoxy-, Hydroxy-, $C_1$-$C_4$-Alkyl- oder Phenoxygruppe
bedeuten, aber nicht gleichzeitig für eine unsubstituierte Arylgruppe stehen, und
der Dritte der Reste $R^3$, $R^4$ und $R^5$ für $-CO_2H$, $-COSC_1$-$C_4$-Alkyl oder A-X steht, wobei A eine geradkettige oder verzweigte $C_1$-$C_8$-Alkylengruppe , die gegebenenfalls durch ein Sauerstoffheteroatom oder eine Carbonylgruppe unterbrochen sein kann, oder eine $C_2$-$C_8$- Alkenylengruppe bedeutet und X für $CO_2H$, $SO_3H$, CHO, oder SH steht, und
$R^6$ und $R^7$ unabhängig voneinander für ein Wasserstoffatom oder eine $C_1$-$C_4$-Alkylgruppe stehen,
sowie deren pharmazeutisch verträgliche Salze und Ester.

2. Verbindungen nach Anspruch 1 der Formel I , worin zwei der Reste $R^3$, $R^4$ und $R^5$ für eine Phenylgruppe, die gegebenenfalls substituiert ist durch ein oder zwei Reste, die ausgewählt sind unter einem Halogenatom, insbesondere ein Fluor- oder Chloratom, einer $C_1$-$C_4$-Alkyl-, $C_1$-$C_4$-Alkoxy-, Hydroxy- Phenoxy- und Nitrogruppe, stehen und der dritte Rest für A-X steht, wobei A eine $C_1$-$C_8$-Alkylengruppe oder eine $C_2$-$C_8$-Alkenylengruppe bedeutet und X für $CO_2H$ steht.

3. Verbindungen nach Anspruch 1 der Formel:

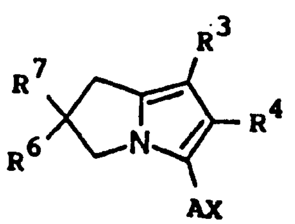

worin
$R^3$ und $R^4$ unabhängig voneinander für eine Phenylgruppe stehen, die durch ein Halogenatom substituiert

sein kann, A für eine $C_1$-$C_8$-Alkylengruppe und X für $CO_2H$ steht.

4. Verbindungen nach Anspruch 3, worin $R^3$ und $R^4$ unabhängig voneinander für eine Phenylgruppe oder p-Chlorphenylgruppe stehen.

5. Verbindungen nach Anspruch 4, worin $R^6$ und $R^7$ für eine $C_1$-$C_4$-Alkylgruppe und insbesondere für eine Methylgruppe stehen.

6. Verfahren zur Herstellung der Verbindungen der Ansprüche 1 bis 5 der Formel I **dadurch gekennzeichnet**, daß man
A) eine Verbindung der allgemeinen Formel II:

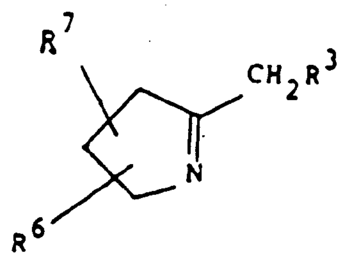

(II)

in der $R^6$ und $R^7$ unabhängig voneinander für ein Wasserstoffatom oder eine $C_1$ - $C_4$-Alkylgruppe stehen,
mit einer Verbindung der allgemeinen Formel III:

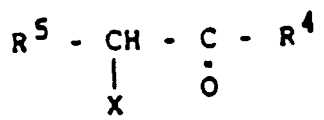

(III)

umsetzt,
worin X für Cl oder Br steht und wobei in diesen Formeln zwei der Reste $R^3$, $R^4$ und $R^5$ unabhängig voneinander die in Anspruch 1 angegebenen Bedeutungen besitzen, und der Dritte der Reste $R^3$, $R^4$ und $R^5$ für ein Wasserstoffatom steht, wobei man eine Verbindung der allgemeinen Formel IV erhält:

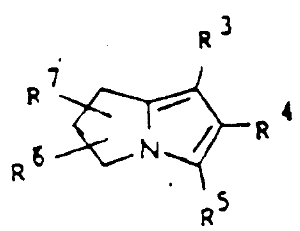

(IV)

worin die Reste $R^3$ bis $R^7$ die genannten Bedeutungen besitzen, und
B) in die so erhaltenen Verbindungen eine Hydroxymethylgruppe, Essigestergruppe, Ameisensäurethioestergruppe oder eine Acylgruppe einführt und diese gewünschtenfalls durch Reduktion, Esterspaltung, Wittig-Reaktion, Hydrierung oder durch eine Kombination dieser Maßnahmen in die gewünschte Bedeutung der Reste $R^3$, $R^4$ oder $R^5$ überführt.

7. Verwendung wenigstens einer Verbindung gemäß einem der Ansprüche 1 bis 5 zur Herstellung eines pharmazeutischen Mittels zur Prävention von allergisch induzierten Erkrankungen und zur Behandlung von Erkrankungen des rheumatischen Formenkreises.

8. Verfahren zur Herstellung eines pharmazeutischen Mittels, dadurch gekennzeichnet, daß man wenigstens eine Verbindung nach einem der Ansprüche 1 bis 5 in einer zur Verabreichung geeigneten Form

bereitstellt, gegebenenfalls in üblichen pharmazeutisch verträglichen Hilfsstoffen und/oder Zusatzstoffen.

## Claims

**Claims for the following Contracting States : AT, BE, CH, DE, DK, FR, GB, IT, LI, LU, NL, SE**

1. Substituted pyrrolizine compounds of the general formula I

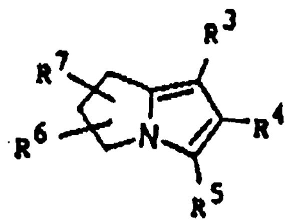

I

in which
two of the radicals $R^3$, $R^4$ and $R^5$ in each case independently of one another denote an aryl group which is optionally substituted by one or two radicals which are selected from amongst a halogen atom, or a nitro, $C_1$-$C_4$-alkoxy, hydroxy, $C_1$-$C_4$-alkyl or phenoxy group but do not simultaneously represent an unsubstituted aryl group, and
the third of the radicals $R^3$, $R^4$ and $R^5$ represents $-CO_2H$, $-COSC_1$-$C_4$-alkyl or A-X, where A denotes a straight-chain or branched $C_1$-$C_8$-alkylene group, which can optionally be interrupted by an oxygen heteroatom or a carbonyl group, or a $C_2$-$C_8$-alkenylene group and X represents $CO_2H$, $SO_3H$, CHO or SH, and
$R^6$ and $R^7$ independently of one another represent a hydrogen atom or a $C_1$-$C_4$-alkyl group,
and their pharmaceutically tolerable salts and esters.

2. Compounds according to Claim 1 of the formula I, in which two of the radicals $R^3$, $R^4$ and $R^5$ represent a phenyl group, which is optionally substituted by one or two radicals which are selected from amongst a halogen atom, in particular a fluorine or chlorine atom, or a $C_1$-$C_4$-alkyl, $C_1$-$C_4$-alkoxy, hydroxy, phenoxy and nitro group, and the third radical represents A-X, where A denotes a $C_1$-$C_8$-alkylene group or a $C_2$-$C_8$-alkenylene group and X represents $CO_2H$.

3. Compounds according to Claim 1 of the formula:

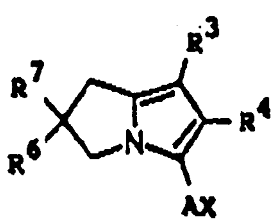

in which $R^3$ and $R^4$ independently of one another represent a phenyl group which can be substituted by a halogen atom, A represents a $C_1$-$C_8$-alkylene group and X represents $CO_2H$.

4. Compounds according to Claim 3, in which $R^3$ and $R^4$ independently of one another represent a phenyl group or p-chlorophenyl group.

5. Compounds according to Claim 4, in which $R^5$ and $R^7$ represent a $C_1$-$C_4$-alkyl group and in particular a methyl group.

6. Pharmaceutical composition, containing at least one compound according to one of Claims 1 to 5, if appropriate together with customary pharmaceutically tolerable auxiliaries and/or additives.

7. Process for the preparation of the compounds of Claims 1 to 5 of the formula I

characterized in that

A) a compound of the general formula II:

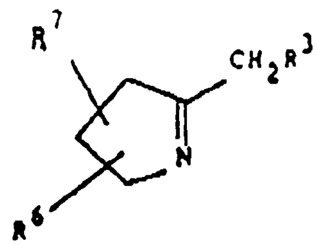

(II)

in which $R^6$ and $R^7$ independently of one another represent a hydrogen atom or a $C_1$-$C_4$-alkyl group, is reacted with a compound of the general formula III:

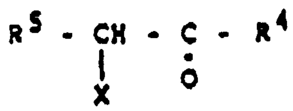

(III)

in which X represents Cl or Br and where in these formulae two of the radicals $R^3$, $R^4$ and $R^5$ independently of one another have the meanings given in Claim 1, and the third of the radicals $R^3$, $R^4$ and $R^5$ represents a hydrogen atom, a compound of the general formula IV being obtained:

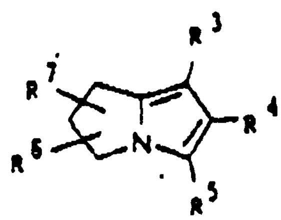

in which the radicals $R^3$ to $R^7$ have the meanings mentioned, and

B) a hydroxymethyl group, acetate ester group, formate thioester group or an acyl group is introduced into the compounds thus obtained and this group is converted, if desired, by reduction, ester cleavage, Wittig reaction, hydrogenation or by a combination of these measures to the desired meaning of the radicals $R^3$, $R^4$ or $R^5$.

8. Use of at least one compound according to one of Claims 1 to 5 for production of a pharmaceutical composition for the prevention of allergically induced disorders and for the treatment of rheumatic disorders.

9. Process for the production of the pharmaceutical composition according to Claim 6, characterized in that at least one compound according to one of Claims 1 to 5 is provided in a form suitable for administration, if appropriate in customary pharmaceutically tolerable auxiliaries and/or additives.

**Claims for the following Contracting State : ES**

1. Process for the preparation of substituted pyrrolizine compounds of the general formula I

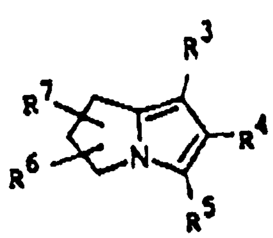

I

in which
two of the radicals $R^3$, $R^4$ and $R^5$ in each case independently of one another denote an aryl group which is optionally substituted by one or two radicals which are selected from amongst a halogen atom, or a nitro, $C_1$-$C_4$- alkoxy hydroxy, $C_1$- $C_4$- alkyl or phenoxy group but do not simultaneously represent an unsubstituted aryl group, and
the third of the radicals $R^3$, $R^4$ and $R^5$ represents -$CO_2H$, -$COSC_1$-$C_4$-alkyl or A-X, where A denotes a straight-chain or branched $C_1$-$C_8$-alkylene group, which can optionally be interrupted by an oxygen heteroatom or a carbonyl group, or a $C_2$-$C_8$-alkenylene group and X represents $CO_2H$, $SO_3H$, CHO or SH, and
$R^6$ and $R^7$ independently of one another represent a hydrogen atom or a $C_1$-$C_4$-alkyl group,
and the pharmaceutically tolerable salts and esters thereof, characterized in that

A) a compound of the general formula II:

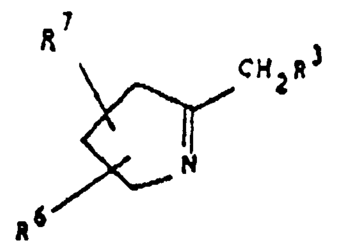

(II)

in which $R^6$ and $R^7$ independently of one another represent a hydrogen atom or a $C_1$-$C_4$-alkyl group, is reacted with a compound of the general formula III:

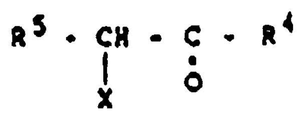

(III)

in which X represents Cl or Br and where in these formulae two of the radicals $R^3$, $R^4$ and $R^5$ independently of one another have the abovementioned meanings, and the third of the radicals $R^3$, $R^4$ and $R^5$ represents a hydrogen atom, a compound of the general formula IV being obtained:

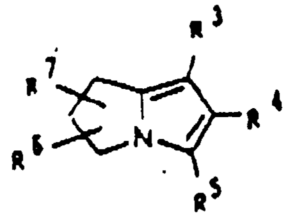

(IV)

in which the radicals $R^3$ to $R^7$ have the meanings mentioned, and
B) a hydroxymethyl group, acetate ester group, formate thioester group or an acyl group is introduced into the compounds thus obtained and this group is converted, if desired, by reduction, ester cleavage, Wittig reaction, hydrogenation or by a combination of these measures to the desired meaning of the radicals $R^3$, $R^4$ or $R^5$.

2. Process according to Claim 1 in which in formula I, two of the radicals $R^3$, $R^4$ and $R^5$ represent a phenyl group, which is optionally substituted by one or two radicals which are selected from amongst a halogen atom, in particular a fluorine or chlorine atom, or a $C_1$-$C_4$-alkyl, $C_1$-$C_4$-alkoxy, hydroxy, phenoxy and nitro group, and the third radical represents A-X, where A denotes a $C_1$-$C_8$-alkylene group or a $C_2$-$C_8$-alkenylene group and X represents $CO_2H$.

3. Process according to Claim 1, in which compounds of the formula:

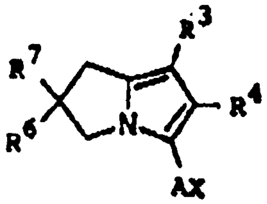

are obtained in which $R^3$ and $R^4$ independently of one another represent a phenyl group which can be substituted by a halogen atom, A represents a $C_1$-$C_8$-alkylene group and X represents $CO_2H$.

4. Process according to Claim 3, in which $R^3$ and $R^4$ independently of one another represent a phenyl group or p-chlorophenyl group.

5. Process according to Claim 4, in which $R^6$ and $R^7$ represent a $C_1$-$C_4$-alkyl group and in particular a methyl group.

6. Process for the preparation of a pharmaceutical composition, characterized in that at least one compound as defined in one of Claims 1 to 5 is provided in a form suitable for administration, if appropriate in customary pharmaceutically tolerable auxiliaries and/or additives.

**Claims for the following Contracting State : GR**

1. Substituted pyrrolizine compounds of the general formula I

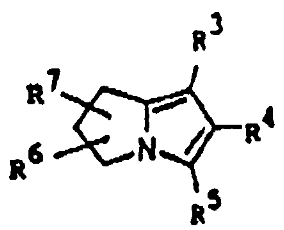

I

in which
two of the radicals $R^3$, $R^4$ and $R^5$ in each case independently of one another denote an aryl group which is optionally substituted by one or two radicals which are selected from amongst a halogen atom, or a nitro, $C_1$-$C_4$-alkoxy, hydroxy, $C_1$-$C_4$-alkyl or phenoxy group but do not simultaneously represent an unsubstituted aryl group, and
the third of the radicals $R^3$, $R^4$ and $R^5$ represents $-CO_2H$, $-COSC_1$-$C_4$-alkyl or A-X, where A denotes a straight-chain or branched $C_1$-$C_8$-alkylene group, which can optionally be interrupted by an oxygen heteroatom or a carbonyl group, or a $C_2$-$C_8$-alkenylene group and X represents $CO_2H$, $SO_3H$, CHO or SH, and
$R^6$ and $R^7$ independently of one another represent a hydrogen atom or a $C_1$-$C_4$-alkyl group,
and their pharmaceutically tolerable salts and esters.

2. Compounds according to Claim 1 of the formula I, in which two of the radicals $R^3$, $R^4$ and $R^5$ represent a phenyl group, which is optionally substituted by one or two radicals which are selected from amongst a halogen atom, in particular a fluorine or chlorine atom, or a $C_1$-$C_4$-alkyl, $C_1$-$C_4$-alkoxy, hydroxy, phenoxy and nitro group, and the third radical represents A-X, where A denotes a $C_1$-$C_8$-alkylene group or a $C_2$-$C_8$-alkenylene group and X represents $CO_2H$.

3. Compounds according to Claim 1 of the formula:

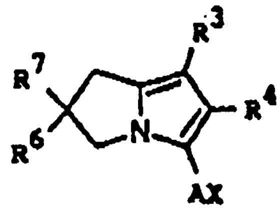

in which $R^3$ and $R^4$ independently of one another represent a phenyl group which can be substituted by a halogen atom, A represents a $C_1$-$C_8$-alkylene group and X represents $CO_2H$.

4. Compounds according to Claim 3, in which $R^3$ and $R^4$ independently of one another represent a phenyl group or p-chlorophenyl group.

5. Compounds according to Claim 4, in which $R^6$ and $R^7$ represent a $C_1$-$C_4$-alkyl group and in particular a methyl group.

6. Process for the preparation of the compounds of Claims 1 to 5 of the formula I characterized in that
A) a compound of the general formula II:

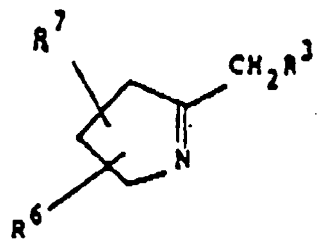

(II)

in which $R^6$ and $R^7$ independently of one another represent a hydrogen atom or a $C_1$- $C_4$- alkyl group, is reacted with a compound of the general formula III:

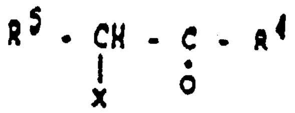

(III)

in which X represents Cl or Br and where in these formulae two of the radicals $R^3$, $R^4$ and $R^5$ independently of one another have the meanings given in Claim 1, and the third of the radicals $R^3$, $R^4$ and $R^5$ represents a hydrogen atom, a compound of the general formula IV being obtained:

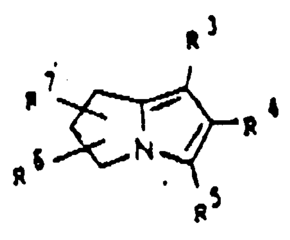

(IV)

in which the radicals $R^3$ to $R^7$ have the meanings mentioned, and
B) a hydroxymethyl group, acetate ester group, formate thioester group or an acyl group is introduced into the compounds thus obtained and this group is converted, if desired, by reduction, ester cleavage, Wittig reaction, hydrogenation or by a combination of these measures to the desired meaning of the radicals $R^3$, $R^4$ or $R^5$.

7. Use of at least one compound according to one of Claims 1 to 5 for production of a pharmaceutical composition for the prevention of allergically induced disorders and for the treatment of rheumatic disorders.

8. Process for the production of a pharmaceutical composition, characterized in that at least one compound according to one of Claims 1 to 5 is provided in a form suitable for administration, if appropriate in customary pharmaceutically tolerable auxiliaries and/or additives.

## Revendications

**Revendications pour les Etats contractants suivants : AT, BE, CH, DE, DK, FR, GB, IT, LI, LU, NL, SE**

1. Dérivés de substitution de la pyrrolizine de formule générale I:

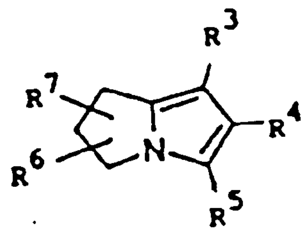

(I)

dans laquelle

chaque fois deux des radicaux $R^3$, $R^4$ et $R^5$ signifient, indépendamment l'un de l'autre, un groupe aryle qui est éventuellement substitué par un ou deux radicaux qui sont choisis parmi un atome d'halogène, un groupe nitro, un groupe $C_1$-$C_4$-alcoxy , un groupe hydroxy , un groupe $C_1$-$C_4$-alcoyle ou un groupe phénoxy, mais ne représentent pas simultanément un groupe aryle qui n'est pas substitué, et

le troisième des radicaux $R^3$, $R^4$ et $R^5$ représente $-CO_2H$, $-COSC_1$-$C_4$-alcoyle ou A-X, étant précisé que A signifie un groupe $C_1$-$C_8$-alcoylène, à chaîne linéaire ou ramifiée, qui peut éventuellement être interrompu par un hétéroatome d'oxygène ou un groupe carbonyle, ou bien signifie un groupe $C_2$-$C_8$-alcénylène et que X représente $CO_2H$, $SO_3H$, CHO, ou SH, et

$R^6$ et $R^7$ représentent, indépendamment l'un de l'autre, un atome d'hydrogène ou un groupe $C_1$-$C_4$ alcoyle,

ainsi que leurs sels et esters pharmaceutiquement compatibles.

2. Dérivés selon la revendication 1 de formule I, dans laquelle deux des radicaux $R^3$, $R^4$ et $R^5$ représentent un groupe phényle, qui est éventuellement substitué par un ou deux radicaux qui sont choisis parmi un atome d'halogène, en particulier un atome de fluor ou un atome de chlore, un groupe $C_1$-$C_4$-alcoyle, un groupe $C_1$- $C_4$-alcoxy un groupe hydroxy, un groupe phénoxy et un groupe nitro, et le troisième radical représente A-X, étant précisé que A signifie un groupe $C_1$-$C_8$-alcoylène ou un groupe $C_2$-$C_8$ -alcénylène et que X représente $CO_2H$.

3. Dérivés selon la revendication 1 de formule:

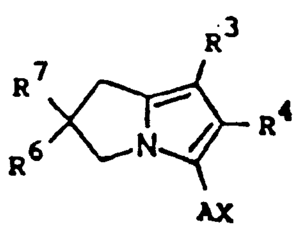

dans laquelle $R^3$ et $R^4$ représentent, indépendamment l'un de l'autre, un groupe phényle qui peut être substitué par un atome d'halogène, A représente un groupe $C_1$-$C_8$-alcoylène et X représente $CO_2H$.

4. Dérivés selon la revendication 3, dans lesquelles $R^3$et $R^4$ représentent, indépendamment l'un de l'autre,

un groupe phényle ou un groupe p-chlorophényle.

5. Dérivés selon la revendication 4, dans lesquelles $R^6$ et $R^7$ représentent un groupe $C_1$-$C_4$-alcoyle et en particulier un groupe méthyle.

6. Produit pharmaceutique contenant au moins un composé selon l'une des revendications 1 à 5, éventuellement avec des adjuvants et/ou des additifs habituels, pharmaceutiquement compatibles.

7. Procédé de préparation des dérivés des revendications 1 à 5, de formule I,
caractérisé par le fait que
A) on fait réagir un composé de formule générale II :

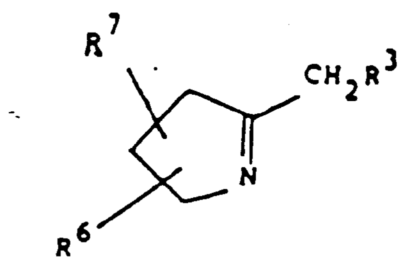

(II)

dans laqelle $R^6$ et $R^7$ représentent, indépendamment l'un de l'autre, un atome d'hydrogène ou un groupe $C_1$-$C_4$-alcoyle,
avec un composé de formule générale III :

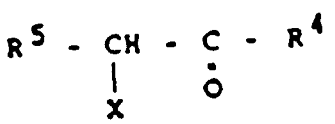

(III)

dans laquelle X représente Cl ou Br et étant précisé que dans ces formules deux des radicaux $R^3$, $R^4$ et $R^5$ possèdent , indépendamment l'un de l'autre, les significations mentionnées dans la revendication 1 et que le troisième des radicaux $R^3$, $R^4$ et $R^5$ représente un atome d'hydrogène, de façon à obtenir un dérivé de formule générale IV :

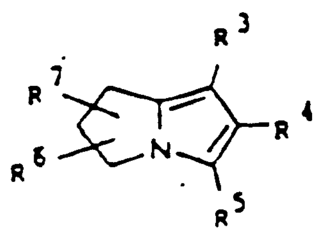

(IV)

dans laquelle les radicaux $R^3$ à $R^7$ possèdent les significations mentionnées et
B) dans les composés ainsi obtenues, on introduit un groupe hydroxyméthyle,
un groupe ester de l'acide acétique, un groupe thioester de l'acide formique ou un groupe acyle et que l'on convertit éventuellement ce groupe en la signification désirée des radicaux $R^3$, $R^4$ ou $R^5$ par réduction, scission de l'ester, réaction de Wittig, hydrogénation ou par une combinaison de ces mesures.

8. Utilisation d'au moins un dérivé selon l'une des revendications 1 à 5 pour obtenir un produit pharmaceutique pour la prévention de maladies provoquées par allergie et pour le traitement des maladies du type rhumatismal.

9. Procédé d'obtention du produit pharmaceutique selon la revendication 6, caractérisé par le fait qu'on prépare au moins un composé selon l'une des revendications 1 à 5 sous une forme appropriée pour administration, éventuellement dans des adjuvants et/ou des additifs habituels, pharmaceutiquement compatibles.

**Revendications pour l'Etat contractant suivant : ES**

1. Procédé de préparation de dérivés de substitution de la pyrrolizine de formule générale I:

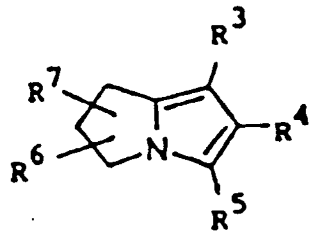

(I)

dans laquelle

chaque fois deux des radicaux $R^3$, $R^4$ et $R^5$ signifient, indépendamment l'un de l'autre, un groupe aryle qui est éventuellement substitué par un ou deux radicaux qui sont choisis parmi un atome d'halogène, un groupe nitro, un groupe $C_1$-$C_4$-alcoxy , un groupe hydroxy, un groupe $C_1$-$C_4$-alcoyle ou un groupe phénoxy, mais ne représentent pas simultanément un groupe aryle qui n'est pas substitué, et

le troisième des radicaux $R^3$, $R^4$ et $R^5$ représente $-CO_2H$, $-COSC_1$-$C_4$-alcoyle ou A-X, étant précisé que A signifie un groupe $C_1$-$C_8$-alcoylène, à chaîne linéaire ou ramifiée, qui peut éventuellement être interrompu par un hétéroatome d'oxygène ou un groupe carbonyle, ou bien signifie un groupe $C_2$-$C_8$-alcénylène et que X représente $CO_2H$, $SO_3H$, CHO, ou SH, et

$R^6$ et $R^7$ représentent, indépendamment l'un de l'autre, un atome d'hydrogène ou un groupe $C_1$-$C_4$ alcoyle,

ainsi que leurs sels et esters pharmaceutiquement compatibles.

procédé caractérisé par le fait que

A) qu'on fait réagir un composé de formule générale II:

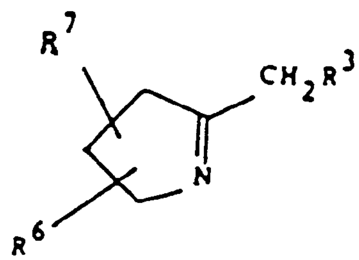

(II)

dans laquelle $R^6$ et $R^7$ représentent, indépendamment l'un de l'autre, un atome d'hydrogène ou un groupe $C_1$-$C_4$-alcoyle,

avec un composé de formule générale III :

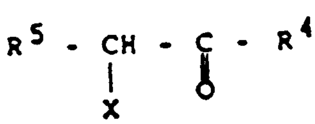

(III)

dans laquelle X représente Cl ou Br et étant précisé que dans ces formules deux des radicaux $R^3$, $R^4$ et $R^5$ présentent, indépendamment l'un de l'autre, les significations données ci-dessus et que le troisième des radicaux $R^3$, $R^4$ et $R^5$ représente un atome d'hydrogène, de façon à obtenir un composé de formule générale IV:

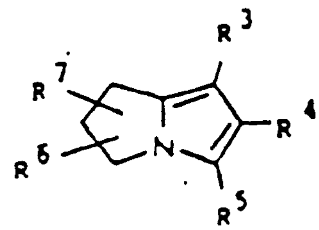

(IV)

dans laquelle les radicaux $R^3$ à $R^7$ possèdent les significations données et

B) dans les composés ainsi obtenus, on introduit un groupe hydroxyméthyle, un groupe ester de l'acide acétique, un groupe thioester de l'acide formique ou un groupe acyle et que l'on convertit éventuellement, ce groupe en la signification désirée des radicaux $R^3$, $R^4$ou $R^5$par réduction, scission de l'ester, réaction de Wittig, hydrogénation ou par une combinaison de ces mesures.

2. Procédé selon la revendication 1, dans lequel, dans la formule I, deux des radicaux $R^3$, $R^4$ et $R^5$ représentent un groupe phényle qui est éventuellement substitué par un ou deux radicaux qui sont choisis parmi un atome d'halogène, en particulier un atome de fluor ou un atome de chlore, un groupe $C_1$-$C_4$-alcoyle, un groupe $C_1$-$C_4$-alcoxy, un groupe hydroxy, un groupe phénoxy et un groupe nitro, et le troisième radical représente A-X, étant précisé que A signifie un groupe $C_1$-$C_8$-alcoylène ou un groupe $C_2$-$C_8$-alcénylène et que X représente $CO_2H$.

3. Procédé selon la revendication 1, dans lequel on obtient des composés de formule :

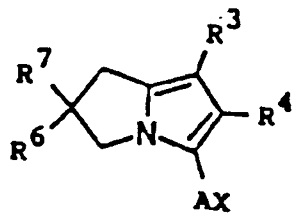

dans laquelle $R^3$ et $R^4$ représentent, indépendamment l'un de l'autre, un groupe phényle qui peut être substitué par un atome d'halogène, A représente un groupe $C_1$-$C_8$-alcoylène et X représente $CO_2H$.

4. Procédé selon la revendication 3, dans lequel $R^3$ et $R^4$ représentent, indépendamment l'un de l'autre, un groupe phényle ou un groupe p-chlorophényle.

5. Procédé selon la revendication 4, dans lequel $R^6$ et $R^7$ représentent un groupe $C_1$-$C_4$-alcoyle et, en particulier, un groupe méthyle.

6. Procédé de préparation d'un produit pharmaceutique, caractérisé par le fait que l'on formule au moins un dérivé selon l'une des revendications 1 à 5 sous une forme appropriée pour administration à un patient, éventuellement dans des adjuvants et/ou des additifs habituels, pharmaceutiquement compatibles.

**Revendications pour l'Etat contractant suivant : GR**

1. Dérivés de substitution de la pyrrolizine de formule générale I:

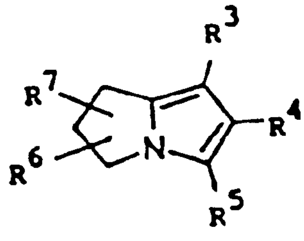

(I)

dans laquelle

chaque fois deux des radicaux $R^3$, $R^4$ et $R^5$ signifient, indépendamment l'un de l'autre, un groupe aryle qui est éventuellement substitué par un ou deux radicaux qui sont choisis parmi un atome d'halogène, un groupe nitro, un groupe $C_1$-$C_4$-alcoxy , un groupe hydroxy, un groupe $C_1$-$C_4$-alcoyle ou un groupe phénoxy, mais ne représentent pas simultanément un groupe aryle qui n'est pas substitué, et

le troisième des radicaux $R^3$, $R^4$ et $R^5$ représente -$CO_2H$, -$COSC_1$-$C_4$-alcoyle ou A-X, étant précisé que A signifie un groupe $C_1$-$C_8$-alcoylène, à chaîne linéaire ou ramifiée, qui peut éventuellement être interrompu par un hétéroatome d'oxygène ou un groupe carbonyle, ou bien signifie un groupe $C_2$-$C_8$-alcénylène et que X représente $CO_2H$, $SO_3H$, CHO, ou SH, et

$R^6$ et $R^7$ représentent, indépendamment l'un de l'autre, un atome d'hydrogène ou un groupe $C_1$-$C_4$ alcoyle,

ainsi que leurs sels et esters pharmaceutiquement compatibles.

2. Dérivés selon la revendication 1 de formule I, dans laquelle deux des radicaux $R^3$, $R^4$ et $R^5$ représentent un groupe phényle qui est éventuellement substitué par un ou deux radicaux qui sont choisis parmi un atome d'halogène, en particulier un atome de fluor ou un atome de chlore, un groupe $C_1$-$C_4$-alcoyle, un groupe $C_1$-$C_4$-alcoxyl, un groupe hydroxy, un groupe phénoxy et un groupe nitro, et le troisième radical représente A-X, étant précisé que A signifie un groupe $C_1$-$C_8$-alcoylène ou un groupe $C_2$-$C_8$-alcénylène et que X représente $CO_2H$.

3. Dérivés selon la revendication 1 de formule:

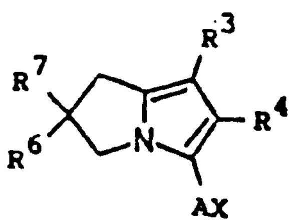

dans laquelle $R^3$ et $R^4$ représentent, indépendamment l'un de l'autre, un groupe phényle qui peut être substitué par un atome d'halogène, A représente un groupe $C_1$-$C_8$-alcoylène et X représente $CO_2H$.

4. Dérivés selon la revendication 3, dans lesquelles $R^3$ et $R^4$ représentent, indépendamment l'un de l'autre, un groupe phényle ou un groupe p-chlorophényle.

5. Dérivés selon la revendication 4, dans lesquelles $R^6$ et $R^7$ représentent un groupe $C_1$-$C_4$ alcoyle et, en particulier, un groupe méthyle.

6. Procédé d'obtention des dérivés des revendications 1 à 5 de formule I,

caractérisée par le fait que

A) on fait réagir un composé de formule générale II :

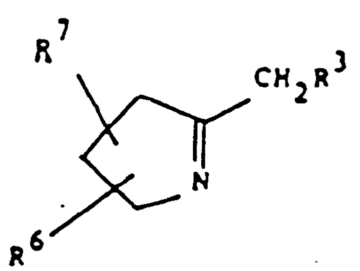

(II)

dans laquelle $R^6$ et $R^7$ représentent, indépendamment l'un de l'autre un atome d'hydrogène ou un groupe $C_1$-$C_4$-alcoyle,

avec un composé de formule générale III :

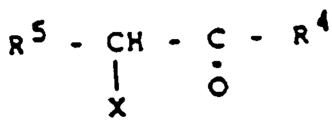

(III)

dans laquelle X représente Cl ou Br et étant précisé que dans ces formules deux des radicaux $R^3$, $R^4$ et $R^5$ possèdent , indépendamment l'un de l'autre, les significations mentionnées dans la revendication 1 et que le troisième des radicaux $R^3$, $R^4$et $R^5$ représente un atome d'hydrogène, de façon à obtenir un dérivé de formule générale IV :

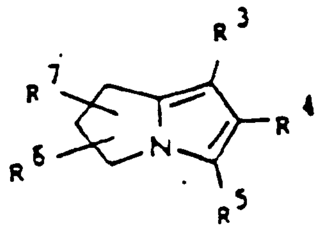

(IV)

dans laquelle les radicaux $R^3$ à $R^7$ possèdent les significations mentionnées et

B) dans les composés ainsi obtenues, on introduit un groupe hydroxyméthyle, méthylol, un groupe ester de l'acide acétique, un groupe thioester de l'acide formique ou un groupe acyle et que l'on convertit éventuellement ce groupe en la signification désirée des radicaux $R^3$, $R^4$ ou $R^5$ par réduction, scission de l'ester, réaction de Wittig, hydrogénation ou par une combinaison de ces mesures.

**7.** Utilisation d'au moins un dérivé selon l'une des revendications 1 à 5 pour obtenir un produit pharmaceutique pour la prévention de maladies provoquées par allergie et pour le traitement des maladies de type rhumatismal.

**8.** Procédé d'obtention d'un produit pharmaceutique, caractérisé par le fait qu'on prépare au moins un dérivé selon l'une des revendications 1 à 5, sous une forme appropriée pour administration à un patient, éventuellement dans des adjuvants et/ou des additifs habituels, pharmaceutiquement compatibles.